# EUROPEAN PATENT APPLICATION

(11) **EP 3 698 797 A1**
(43) Date of publication of application: **26.08.2020**
(21) Application number: 18867486.5
(22) Date of filing: 15.10.2018
(51) Int. Cl.: A61K 31/675, A61K 31/366, A61P 35/00

(54) **MEVALONIC ACID PATHWAY INHIBITOR AND PHARMACEUTICAL COMPOSITION THEREOF**

(30) Priority: 16.10.2017 CN 201710962422
(71) Applicant: Tsinghua University, Beijing 100084 (CN)
(72) Inventor: ZHANG, Yonghui, Beijing 100084 (CN); ZHOU, Xiaoying, Beijing 100084 (CN); XIAO, Hongying, Beijing 100084 (CN); XIA, Yun, Beijing 100084 (CN); XIE, Yonghua, Beijing 100084 (CN); YU, Zhengsen, Beijing 100084 (CN)
(74) Representative: Maiwald Patent- und Rechtsanwaltsgesellschaft mbH
(86) International application number: PCT/CN2018/110256
(87) International publication number: WO 2019/076269

(57) **Abstract**

Provided in the present invention is the use of a mevalonic acid pathway inhibitor in the preparation of a drug for treating and/or preventing tumours, inducing immunogenic death of tumour cells, inducing immune responses and activating T cells or DC cells to directly promote host immunity. Also provided in the present invention is a pharmaceutical composition comprising the mevalonic acid pathway inhibitor and an antitumour drug (for example, an immune checkpoint inhibitor and any drug that can lead to new antigen production) and a combination of the mevalonic acid pathway inhibitor and an antitumour drug (for example, an immune checkpoint inhibitor and any drug that can lead to new antigen production) and/or the radiotherapy.

## Description

### TECHNICAL FIELD

The present disclosure relates to a mevalonate pathway inhibitor that can cause immunogenic cell death of tumor cells, induce an immune response in the body, and directly promote immunity of the host body, and to a use of a mevalonate pathway inhibitor in the manufacture of a medicament for causing tumor cell immunogenic cell death, inducing immune response of the body and directly promoting immunity of the host body. The present disclosure also relates to a pharmaceutical composition of a mevalonate pathway inhibitor and an antitumor drug (such as an immune checkpoint inhibitor and any drug capable of causing neo-antigen production), which have a synergistic antitumor effect, and to a use of the pharmaceutical composition in the manufacture of a medicament for treating tumors. The disclosure also relates to a combination of a mevalonate pathway inhibitor with an antitumor drug (such as immune checkpoint inhibitors and any drug capable of causing neo-antigen production) or a radiation therapy, which have a synergistic antitumor effect, and to a use of the combination in the manufacture of a medicament for treating tumors. The disclosure also relates to activated DC cells, a tumor vaccine comprising said cells, and adoptive DC cells treatment.

### BACKGROUND

The mevalonate (MVA) pathway is an important pathway for the basic metabolism of cells. Similarly, tumor cells rely on the MVA pathway to maintain their unlimited proliferation. Recent studies have found that the activation of tumor proto-oncogenes such as MYC or the inactivation of tumor suppressor genes such as TP53 affects the MVA pathway, making the MVA pathway in tumor cells more active than that in normal cells. Therefore, targeting the MVA pathway is also a strategy for cancer treatment.

The mevalonate pathway is a metabolic pathway for the synthesis of isopentenyl pyrophosphate (IPP) and dimethylallyl pyrophosphate (DMAPP) from acetyl-CoA as a raw material and is present in all higher eukaryotes and many viruses. The product of this pathway can be thought of as an activated isoprene unit, which is a synthetic precursor of steroids, terpenoids and other biomolecules. In this pathway, acetoacetyl-CoA is produced by two molecules of acetyl-CoA, and the resulting acetoacetyl-CoA is then reacted with acetyl-CoA to produce 3-hydroxy-3-methylglutaryl CoA, i.e., HMG-CoA, and then HMG-CoA is reduced to mevalonate under the action of HMG-CoA reductase. The mevalonate is catalyzed by two kinases and one decarboxylase to form isopentenyl pyrophosphate (IPP). Under the catalysis of FPP synthase (FPPS), IPP forms farnesyl pyrophosphate (FPP). FPP forms in different downstream pathways, for example cholesterol, ubiquinone, Heme A, sterol, dolichol and prenylated proteins. For example, FPP can form squalene under the action of squalene synthase (SQS), and squalene produces cholesterol under the catalysis of a series of enzymes. Under the action of farnesyl transferase, FPP is able to perform farnesylation modification to some proteins. On the other hand, under the catalysis of GGPP synthase (GGPPS), FPP affords geranylgeranyl pyrophosphate (GGPP), whereas under the action of geranylgeranyl transferase, GGPP is capable of carrying out geranylgeranylation modification on some proteins to form prenylated proteins.

Specifically, mevalonate pathway inhibitors include the following types: 1) Thiolase (acetoacetyl-CoA transferase) inhibitors; 2) HMG-CoA synthase inhibitors; 3) HMG-CoA reductase inhibitors, such as statins; 4) Mevalonate kinase inhibitors; 5) Phosphonomevalonate kinase inhibitors; 6) Mevalonate-5-pyrophosphate decarboxylase inhibitors; 7) Isopentenyl-diphosphate isomerase inhibitors; 8) Farnesyl diphosphate synthase (FPPS) inhibitors, such as bisphosphonates drugs; 9) Geranylgeranyl diphosphate synthase (GGPPS) inhibitors; and 10) Geranylgeranyl transferase (I, II) inhibitors.

Both statins and bisphosphonates have been found to be effective against multiple tumor types (The interplay between cell signalling and the mevalonate pathway in cancer, Nat Rev Cancer. 2016 Nov; 16(11):718-731; Direct and indirect anticancer activity of bisphosphonates: A brief review, Cancer Treat Rev. 2012 Aug; 38(5):407-15). Bisphosphonates, taking zoledronic acid as an example, has direct or indirect antitumor effects, and its antitumor effects are mainly manifested in promoting tumor cells apoptosis, inhibiting the extracellular matrix adhesion of tumor cells, inhibiting tumor metastasis and invasion, resisting angiogenesis, activating and amplifying γδ T of the host, and so on. The research is mainly focused on breast cancer, prostate cancer, lung cancer, and multiple myeloma, etc. In addition, data from multiple meta-analysis found that taking bisphosphonate can reduce the risk of occurrence of various tumors such as breast cancer and the bone metastasis (Adjuvant bisphosphonate treatment in early breast cancer: meta-analyses of individual patient data from randomised trials, Lancet. 2015 Oct 3; 386(10001):1353-1361), prostate cancer, lung cancer, etc. The direct antitumor effects of statins are mainly inhibiting tumor cells proliferation and promoting tumor cells apoptosis. Similarly, data from multiple meta-analysis showed that taking statins can reduce the risk of breast cancer relapse in patients (Statins and cancer: a systematic review and meta-analysis, Eur J Cancer. 2008 Oct; 44(15):2122-32), and reduce the risk of hepatocellular carcinoma, prostate cancer, colorectal adenoma, etc. The direct antitumor mechanism of the both drugs is to eventually block the prenylation of GTP-binding proteins such as Rho, Ras or Rac, so that these proteins cannot be anchored to the cell membrane to play their biological role. However, the clinical dose of zoledronic acid is about 4-5 mg per year, which low dose has limited direct antitumor effect. Therefore, mevalonate pathway inhibitors such as zoledronic acid may have other mechanisms to reduce the risk of recurrence of multiple tumors.

Immunogenic cell death (ICD) refers to the release of a dead cell antigen during cell death (especially when tumor cells die), which antigen can stimulate the body's immune response (Immunogenic and tolerogenic cell death, Nat Rev Immunol. 2009 May; 9(5): 353). The concept of ICD is from clinic based on the tumor-specific immune response of tumor patients after taking some conventional chemotherapeutic drugs (Immune parameters affecting the efficacy of chemotherapeutic regimens, Nat Rev Clin Oncol. 2011 Mar; 8(3): 151-60). Chemotherapeutic drugs such as anthracyclines and oxaliplatin, which can trigger ICD, can change the immunosuppressive tumor microenvironment, increase the infiltration of CD8 + T lymphocytes in the tumor, and reduce the number of FOXP3 + Treg. It explains why anthracyclines and oxaliplatin have better antitumor effects on immunocompetent mouse tumor models than on immunodeficient mouse tumor models. In addition to directly killing tumor cells, anthracyclines promote tumors to release neo-antigen or type I interferon signals, which are then phagocytosed, processed, and signal-presented by dentritic cells (DC) to trigger a series of immune responses (Cancer cell-autonomous contribution of type I interferon signaling to the efficacy of chemotherapy, Nat Med. 2014 Nov; 20(11): 1301-9.

### SUMMARY OF THE INVENTION

In the present disclosure, for the first time, we have discovered and confirmed that mevalonate pathway inhibitors such as statins and bisphosphonates can induce tumor cell immunogenic cell death, induce the immune response of the body, and directly promote the immunity of the host body, and to exert better antitumor effects in combination with antitumor drugs (such as immune checkpoint inhibitors such as PD-1 antibodies, nucleoside chemotherapeutics such as 5-FU) and the radiation therapy.

We found that mevalonate pathway inhibitors such as statins and bisphosphonates have the following antitumor mechanisms: 1. statins and bisphosphonates induce tumor cell immunogenic cell death (ICD), thereby activating the immune system of the host: promoting tumor cells membrane to express the "eat me" signaling molecules, calreticulin and ERp57, making the tumor cell easier for priming T cells after phagocytosed by DC cells; promoting tumor cells to express type I interferon (IFN-I) and type I interferon-activated gene ISG; increasing the number of tumor-infiltrating CD8 + T cells, CD4 + T cells, and DC cells in mouse tumor models of homologous transplantation; and using drug-treated tumor cells as tumor vaccines to protect the body against the re-challenge of tumor cells; 2. mevalonate pathway inhibitors directly promote DC immune response: statins and bisphosphonates promote the cross-priming of T cells by DC cells; statin compounds and bisphosphonates promote adoptive DC cells infusion to treat tumors; 3. antitumor effects of statins and bisphosphonates depend on host immune system including T cells and DC cells; 4. combined with PD-1 antibody, chemotherapeutic drug 5-FU and the radiation therapy to have a synergistic antitumor effect.

In one aspect, the disclosure relates to the use of a mevalonate pathway inhibitor in the manufacture of a medicament for the treatment and/or prevention of tumors. The disclosure also relates to the use of a mevalonate pathway inhibitor in the manufacture of a medicament for inducing immunogenic cell death of tumor cells to induce immune responses. The disclosure also relates to the use of a mevalonate pathway inhibitor in the manufacture of a medicament for activating DC cells to directly promote the immunity of the host body. The disclosure also relates to the use of a mevalonate pathway inhibitor in the manufacture of a medicament for activating T lymphocytes and promoting immunity of the host body.

In another aspect, the disclosure relates to a pharmaceutical composition comprising a mevalonate pathway inhibitor and an antitumor drug (such as an immune checkpoint inhibitor and any drug capable of causing neo-antigen production).

In another aspect, the disclosure relates to the use of a mevalonate pathway inhibitor and an antitumor agent (such as an immune checkpoint inhibitor and any drug capable of causing neo-antigen production) in the manufacture of a medicament for treating tumors.

In another aspect, the disclosure relates to a combination of drugs comprising (1) a mevalonate pathway inhibitor, and (2) an antitumor drug (such as an immune checkpoint inhibitor and any drug capable of causing neo-antigen production) or the radiation therapy.

In another aspect, the disclosure relates to a method of treating a tumor, the method comprising administering to a subject in need thereof: (1) a mevalonate pathway inhibitor, and (2) an anti-tumor drug (e.g., immune checkpoint inhibitors and any drug capable of causing neo-antigen production) or the radiation therapy.

In another aspect, the disclosure relates to a mevalonate pathway inhibitor, for use in combination with an antitumor drug (such as an immune checkpoint inhibitor and any drug capable of causing neo-antigen production) or a radiation therapy for the treatment of tumors.

In another aspect, the disclosure relates to a method of treating a tumor, said method comprising infusing DC cells into a subject in need thereof, wherein said DC cells have been treated with a mevalonate pathway inhibitor; preferably, said DC cells are donor DC cells; preferably, the DC cells are autologous DC cells.

In another aspect, the disclosure relates to a method of activating DC cells, said method comprising treating said DC cells with a mevalonate pathway inhibitor.

In another aspect, the disclosure relates to activated DC cells that have been treated with a mevalonate pathway inhibitor.

In another aspect, the disclosure relates to a tumor vaccine comprising activated DC cells which have been treated with a mevalonate pathway inhibitor.

The mevalonate pathway inhibitor and immune checkpoint inhibitor according to the present disclosure are described in detail below.

### BRIEF DESCRIPTION OF THE DRAWINGS

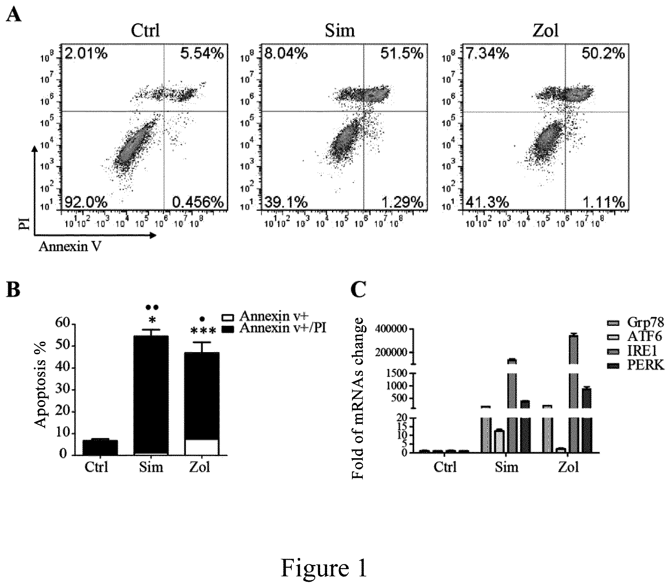

Figure 1 shows that simvastatin and zoledronic acid induce apoptosis and endoplasmic reticulum stress. (Figure 1A) Representative figures of flow cytometry of simvastatin and zoledronic acid induced the apoptosis of B16F10 cells. 1×10⁵ B16F10 cells were seeded in a 96-well plate, which were treated by drugs after adhesion overnight: blank control group; Sim, 2 µM for 24 h; Zol, 10 µM for 48 h. After the drug action, Annexin V and PI staining were performed. (Figure 1B) Quantitative diagrams of the data in Figure 1A, which show that after the treatment with Sim and Zol, the percentage of cells with Annexin + PI + late stage of apoptosis increased significantly. (Figure 1C) B16F10 cells treated with Sim and Zol overexpressed genes related to endoplasmic reticulum stress. B16F10 cells were treated with 10 µM Sim and Zol for 60 h. * and •: P <0.05, ** and •: P <0.01, ***: P <0.001 (* for Ann +, • for Ann + PI +).

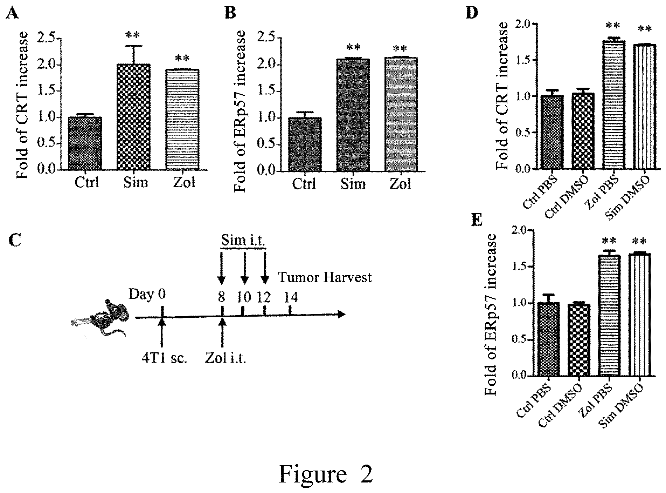

Figure 2 shows that simvastatin and zoledronic acid induce tumor cells to express CRT and ERp57. (Figures 2A and 2B) 1 × 10⁵ B16F10 cells were seeded in a 6-well plate, and treated with Sim 10 µM for 24 h and Zol 20 µM for 60 h. After the drug treatment, the expression of CRT and ERp57 on the cell membrane surface was detected by a flow cytometer. (Figure 2A) The expression of CRT on the surface of B16F10 cell membrane. (Figure 2B) The expression of ERp57 on the surface of B16F10 cell membrane. (Figure 2C) Flow chart of the in vivo experiment. A 4T1 subcutaneous mouse model was used, and the mice were injected intratumorally with Zol 2 mg/kg and Sim 1 mg/kg. (Figure 2D) At the end of the experiment as shown in Figure 2C, the tumor was dissociated into single cells with a tumor dissociation kit for staining, and detected for the CRT expression on the surface of the cell membrane. (Figure 2E) At the end of the experiment as shown in Figure 2C, the tumor was dissociated into single cells with a tumor dissociation kit for staining, and detected for the expression of ERp57 on the surface of the cell membrane. **: P <0.01.

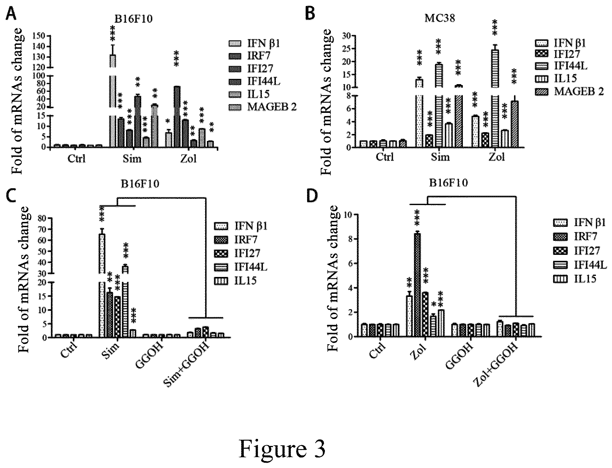

Figure 3 shows the expression of IFNβ1 and ISG genes in tumor cells after the drug treatment. B16F10 cells and MC38 cells were seeded in 6-well plates with a density of 1 × 10⁵ cells per well, respectively, and treated with Sim 10 µM and Zol 10 µM (Figure 3A) for 72 h, or Sim 10 µM and Zol 10 µM together with GGOH 20 µM simultaneously for 48 h (Figures 3B, 3C). Cellular RNA was extracted after the drug action was completed, and the gene expression was detected by a Q-PCR method. (Figure 3A, 3B) After Sim and Zol treatment, the IFN β1 and other ISG genes IRF7, IFI27, IFI44L, IL15, and MAGEB2 expressed by B16F10 cells and MC38 cells. (Figure 3C) After GGOH and Sim simultaneously treatment, the IFN β1and other ISG genes expressed by B16F10 cells. (Figure 3D) After GGOH and Zol simultaneously treatment, the IFN β1and other ISG genes expressed by B16F10 cells. *: P <0.05, **: P <0.01, ***: P <0.001.

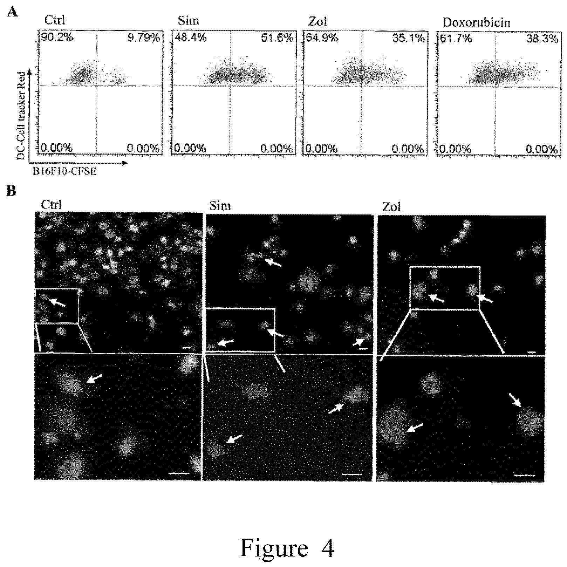

Figure 4 shows that B16F10 cells treated with simvastatin and zoledronic acid are more easily phagocytosed by DC cells. B16F10 cells were treated with Sim 30 µM for 12 h; Zol 30 µM for 24 h; and Doxorubicin 10 µM for 12 h, and blank control was set, which were co-cultured with the same number of DC cells for 4 h to observe the DC phagocytosis. (Figure 4A) Representative figures of flow cytometry, wherein B16F10 cells were stained with CFSE (green), and DC cells were stained with Cell tracker Red (red). (Figure 4B) Representative confocal microscopy photographs, wherein DC cells were stained with CFSE (green), B16F10 cells were stained with Cell tracker Red (red), and the white arrows indicate that the green DC cells have swallowed the red B 16F10 cells with a scale of 20 µm.

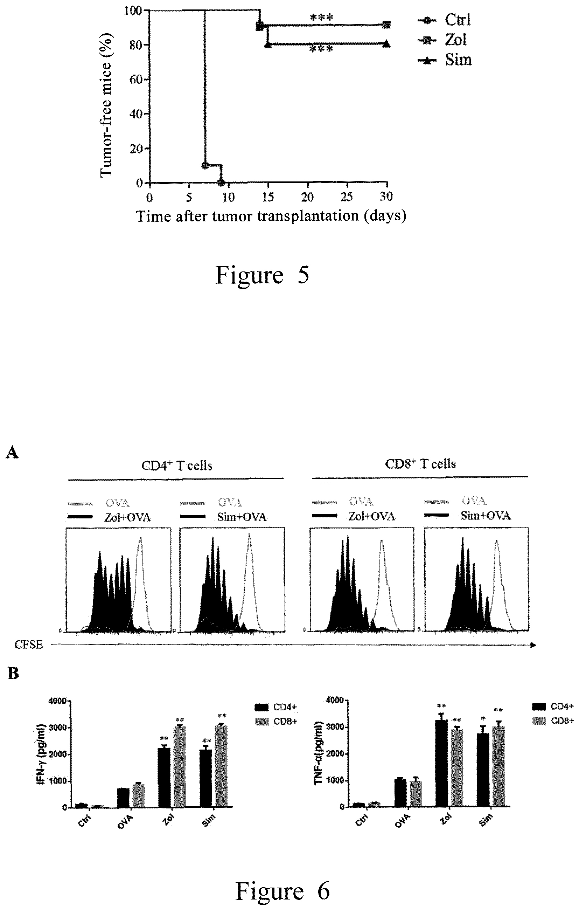

Figure 5 shows that CT26 cells treated with simvastatin and zoledronic acid can withstand the re-challenge of live CT26 cells. CT26 mouse colon cancer cells were treated with Sim 10 µM and Zol 30 µM for 72 h. After the treatment, the cells were in a dying state. These dying cells were transplanted subcutaneously into the left hip of BALB/c mice at 1 × 10⁵ cells per mouse. The control group was injected with PBS. After 7 days, 5 × 10⁵ live CT26 cells were injected into the right hip of each mouse. The tumor growth was observed after 7 days, wherein a tumor volume greater than 100 mm³ stands for the tumor growth. The number of the tumor free mice was recorded. The number of mice in each group was set to n = 10. ***: P <0.001.
Figure 6 shows that Sim and Zol promote the cross-activation of DC cells which cause T cell proliferation and the release of cytokines IFN-γ and TNF-α. BMDC was obtained from the differentiation culture of BALB/c mouse bone marrow cells. BMDC cells were treated with the culture medium control, Sim 1 µM and Zol 5 µM for 24 h, and the medium was washed away after the drug action, and ready to use. The spleen cells of OT-I and OT-II transgenic mice were sorted with a CD4 and a CD8a magnetic beads to obtain CD4 cells and CD8 cells, respectively, and stained with CFSE. After that, 5 × 10⁴ CFSE-stained CD4 cells and CD8 cells were co-cultured with 5 × 10⁴ BMDC cells under OVA (100 µg/mL) for 72 h. (Figure 6A) CD4 +, CD8 + cell proliferation. Cells were stained with CD3 + CD4 + and CD3 + CD8 + antibodies, respectively, and cells were detected by flow cytometer for CFSE to determine their proliferation. (Figure 6B) The secretion of IFN-γ and TNF-α in the supernatant after culture. *: P <0.05, **: P <0.01 (control group is OVA-treated group).

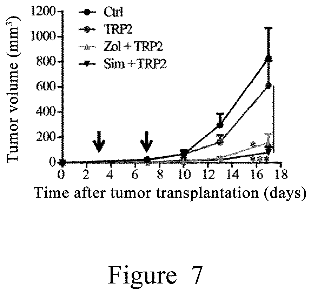

Figure 7 shows that simvastatin and zoledronic acid promote adoptive transfer of DC cells to treat tumors. 2.5 × 10⁵ B16F10 cells were implanted subcutaneously into the left side of C57BL/6 mice, and on the 3rd and 7th day after tumor implantation, 1 × 10⁶ DC cells were injected into the left footpads respectively. Mice were divided into 4 groups according to the DC cell treatment methods: control group, DC cells were not treated; TRP2 group, DC cells were incubated with 50 µg/ml TRP2 protein for 8 h; Zol + TRP2 group, DC cells were first incubated with 5 µM Zol for 24 h, and then with 50 µg/ml TRP2 protein for 8 h; Sim + TRP2 group, DC cells were first incubated with 1 µM Sim for 24 h, and then with 50 µg/ml TRP2 protein for 8 h. Tumor volume was measured every 3-4 days after two DC cell immunizations. *: P <0.05, ***: P <0.001.

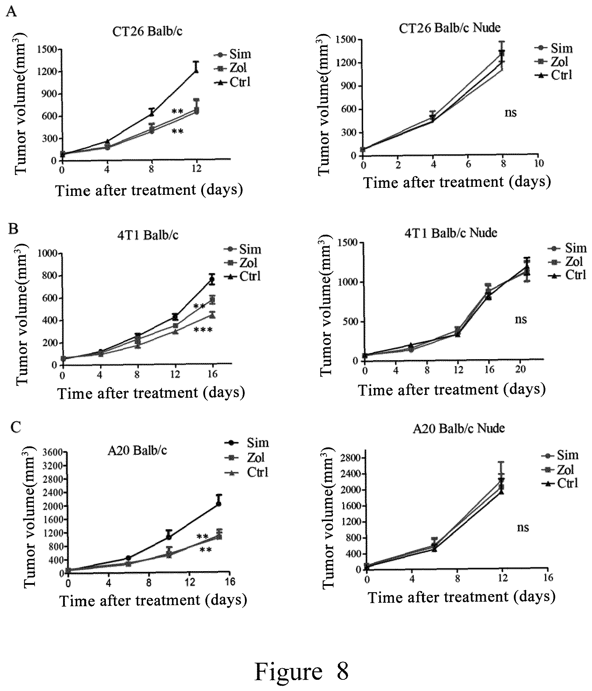

Figure 8 shows the antitumor effect of simvastatin and zoledronic acid in BALB/c mice and BALB/c nude mice. (Figure 8A) Antitumor effect of Zol and Sim in BALB/c mice and BALB/c nude mice transplanted with CT26 cells subcutaneously. (Figure 8B) Antitumor effect of Zol and Sim in BALB/c mice and BALB/c nude mice transplanted with 4T1 cells subcutaneously. (Figure 8C) Antitumor effect of Zol and Sim in BALB/c mice and BALB/c nude mice transplanted with A20 cells subcutaneously. **: P <0.01, ***: P <0.001, ns: No significant difference.

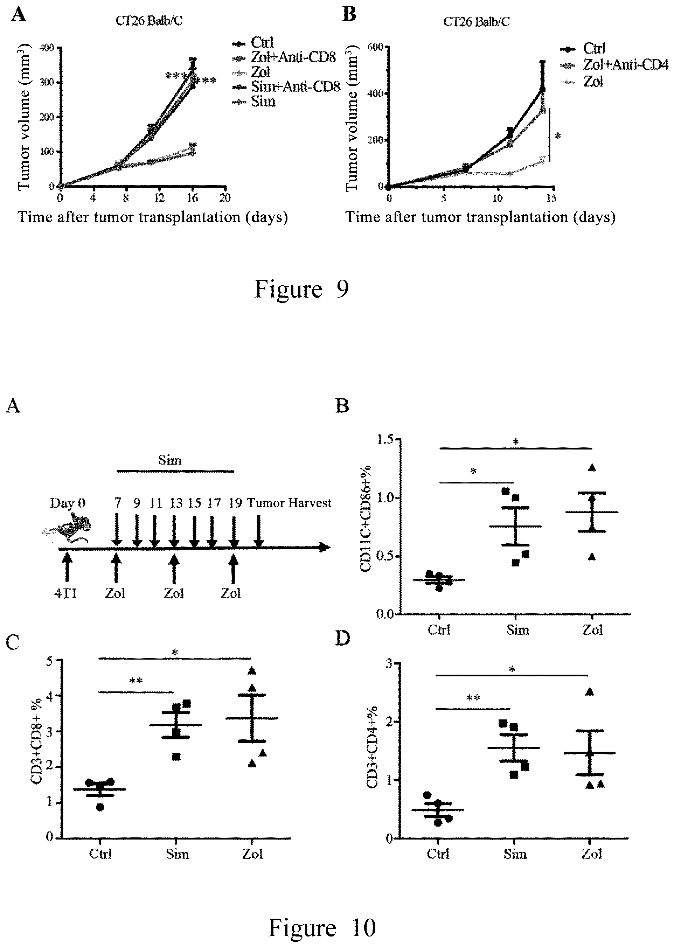

Figure 9 shows that the antitumor effect of zoledronic acid and simvastatin is depend on CD8 T cells and CD4 T cells. 1x10⁶ CT26 cells were seeded subcutaneously into the left side of female, 6-weeks old, BALB/c mice, and the mice were treated (i.p.) group by group starting on day 7 of implantation. Tumor volume was measured every 3 to 4 days. (Figure 9A) CD8 neutralizing antibodies counteracted the antitumor effect of Zol and Sim. (Figure 9B) CD4 neutralizing antibodies counteracted the antitumor effect of Zol. *: P <0.05, ***: P <0.001.
Figure 10 shows that simvastatin and zoledronic acid promote lymphocyte infiltration in tumors. Figure 10A shows the experimental flowchart. Figure 10B shows the percentage of CD45-FITC/CD11c-APC/CD86-PE positive cells to total cells in the tumor for each group. Figure 10C shows the percentage of CD45-FITC/CD3-APC/CD8PE positive cells to total cells in the tumor for each group. Figure 10D shows the percentage of CD45-FITC/CD3-APC/CD4-PE positive cells to total cells in the tumor for each group. *: P <0.05, **: P <0.01.

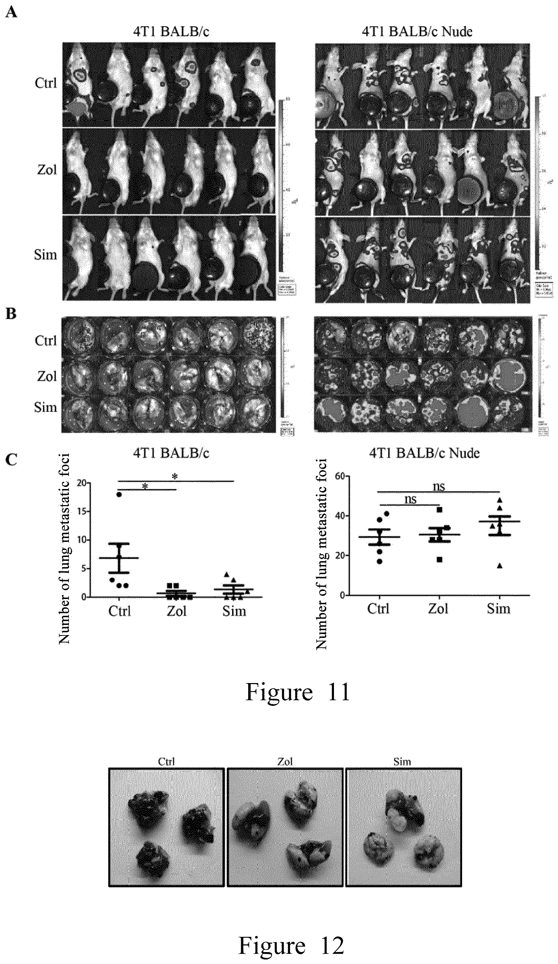

Figure 11 shows the effect of simvastatin and zoledronic acid against tumor metastasis is dependent on T lymphocytes. 40 µl of Luci-4T1 cells containing 5 x 10⁵ cells were injected into the fourth pair of left breast fat pads. Seven days after the cells were injected, the tumor tissues with the size of a soybean can be sensed by touching, and the administration was started in groups. Dosing plan: control group: equal volume of PBS or 75% DMSO; zoledronic acid administration group: 1 mg/kg, once every 6 days, BALB/c mice were administered 4 times in total, BALB/c nude mice were administered 3 times in total; simvastatin administration group: 2 mg/kg, once every two days, BALB/c mice were administered a total of 13 times, and BALB/c nude mice were administered a total of 10 times. BALB/c mice and BALB/c nude mice were injected with Luciferin substrate on the 28th and 21st days after the administration, respectively. After 10 minutes of the injection, the tumor metastasis was photographed with an in-vivo imager, and a black cover was used to cover the tumor in situ. After taking photos, the mice were euthanized. The lungs of each group of mice were removed, and the Luciferin substrate was continuously added. The lung metastasis was shown by imaging. (Figure 11A) The in-vivo imager showed the situation of systemic metastasis of tumor in BALB/c mice and BALB/c nude mice for the control group, simvastatin and zoledronic acid administration groups. (Figure 11B) The in-vivo imager showed the situation of lung metastasis in isolated lungs. (Figure 11C) Number of metastatic foci in isolated lungs. *: P <0.05.
Figure 12 shows that simvastatin and zoledronic acid inhibit the lung metastasis of B 16F10 cells. 3 × 10⁵ B 16F10 cells were resuspended in 100 µl of Opti-MEM and injected into the tail vein of BALB/c mice. Seven days after the injection, the mice were randomly divided into groups and administered. Dosing plan: control group: equal volume of PBS or 75% DMSO; Zol administration group: 1 mg/kg, once every 6 days for a total of 4 times; Sim administration group: 2 mg/kg, once every two days for a total of 8 times. On the 25th day after administration, the mice were euthanized (CO2 method), the lungs were removed, and the metastasis situation of B16F10 cells in lungs was photographed and recorded.

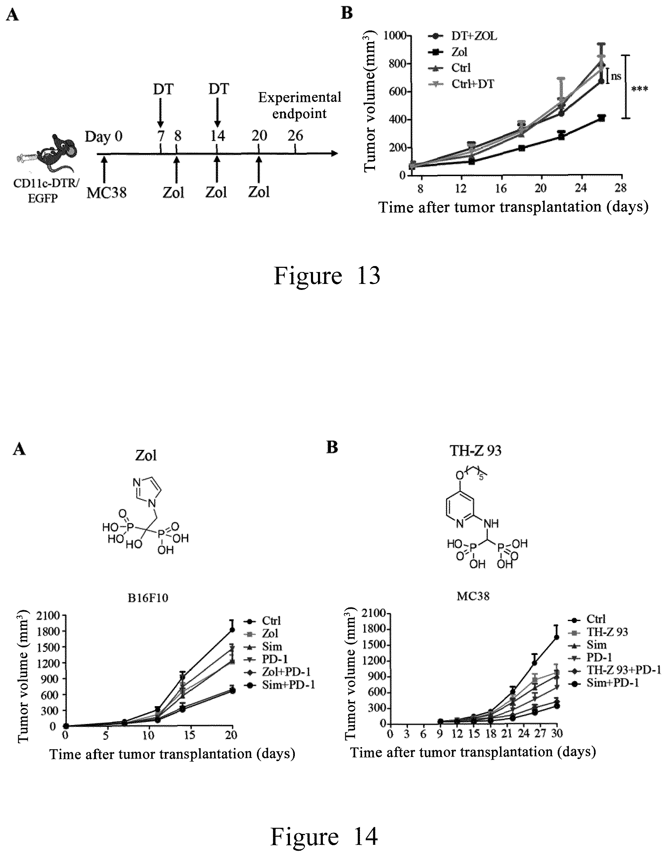

Figure 13 shows that the effect of zoledronic acid against tumor metastasis is dependent on DC cells. 1 × 10⁶ MC38 cells were resuspended in 100 µl Opti-MEM and injected subcutaneously into the left side of 10-week-old female CD11c-DTR/EGFP mice (DTR, diphtheria toxin receptor). The tumor volume was measured on day 7 after implantation, and then the mice were evenly divided into groups and administration was started. (Figure 13A) Experimental flowchart. (Figure 13B) Antitumor effects of Zol, Zol plus DT (diphtheria toxin). ***: P <0.001.
Figure 14 shows that Sim, Zol, and TH-Z 93 have a combined antitumor effect with PD-1 antibodies. 1x10⁶ B16F10 and MC38 cells were inoculated subcutaneously into the left side of 6-week-old female BALB/c mice, respectively. Grouping and administration were started on the 7th and 9th days after tumor transplantation. Zol and Sim were administered intraperitoneally (i.p.). The tumor volume was measured using a vernier caliper, and the tumor volume was calculated using the formula of length (mm) × width² (mm)/2. Tumor volume was measured every 3 to 4 days. Dosing regimen for B16F10 subcutaneous model: Zol, 1 mg/kg, once every 6 days for a total of 3 times; Sim, 2 mg/kg, once every two days for a total of 7 times; PD-1, 200 µg/mouse, once every 3 days for a total of 5 times; the dose of the combined group was the sum of two single doses. Dosing regimen for MC38 subcutaneous model: TH-Z 93, 1 mg/kg, once every 6 days for a total of 4 times; Sim, 2 mg/kg, once every two days for a total of 11 times; PD-1, 200 µg/mouse, once every 3 days for a total of 5 times; the dose of the combined group was the sum of two single doses. (Figure 14A) Antitumor effect of Sim, and Zol in combination with PD-1 antibodies in a subcutaneous tumor model of B16F10 mice. The endpoint of the experiment: Zol + PD-1, CDI = 0.69; Sim + PD-1, CDI = 0.68. (Figure 14B) Antitumor effect of Sim, and TH-Z 93 in combination with PD-1 antibodies in MC38 mouse subcutaneous tumor model. TH-Z 93 + PD-1, CDI = 0.9, Sim+ PD-1, CDI = 0.8.

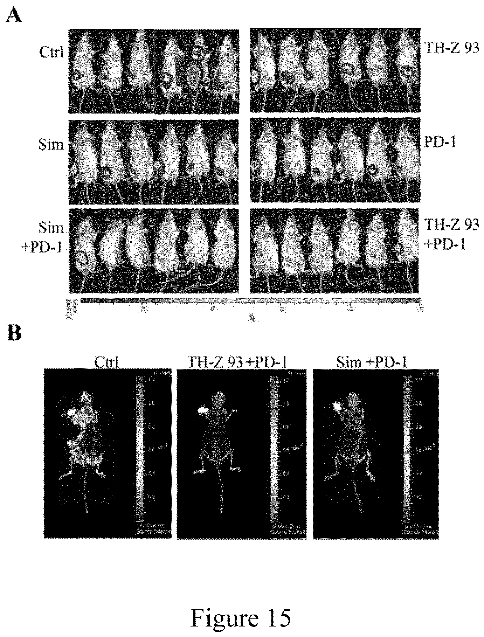

Figure 15 shows the combined antitumor effect of Sim, and TH-Z 93 with PD-1 antibodies (in-vivo imaging). 5x10⁵ Luci-4T1 cells were injected into the breast fat pad. Seven days after the cells were injected, the tumor tissues with the size of a soybean could be sensed by touching. The tumor size was quantified by in-vivo imaging, and grouping and administration was started. Principle of grouping: mean values of tumor chemiluminescence for mice in all groups are equal. Dosing regimen: control group: equal volume of PBS or 75% DMSO; TH-Z 93 administration group: 1 mg/kg, once every 6 days for a total of 3 times; Sim group: 2 mg/kg, once every two days for a total of 11 times; PD-1, 200 µg/mouse, once every 3 days for a total of 7 times; the combined group dose was the sum of two single doses. The mice were subjected to 2D chemiluminescence imaging on the 23rd day after dosing, and to the photographing by Quantum FX Micro CT in-vivo imaging system in combination with Micro CT locating to determine the tumor metastasis on the 31st day. (Figure 15A) Tumor chemiluminescence of mice in each group after in-vivo imaging on day 23 after administration. (Figure 15B) Representative photos of mice in the control group, TH-Z 93 + PD-1 group, and Sim + PD-1 group for Luciferin luminescence imaging combined with Micro CT imaging on the 31st day after administration.

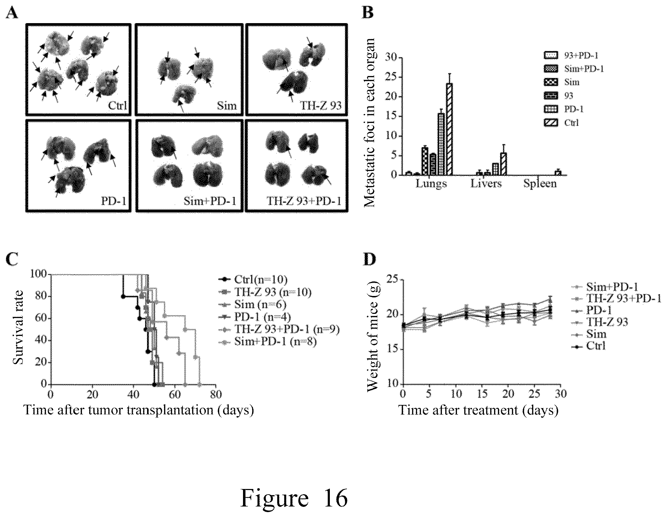

Figure 16 shows that the combination of Sim, and TH-Z 93 with PD-1 antibody inhibits tumor metastasis and improves survival rate of tumor-bearing mice. 5x10⁵ Luci-4T1 cells were injected into the breast fat pad. Seven days after the cells were injected, the tumor tissues with the size of a soybean could be sensed by touching. The tumor size was quantified by in-vivo imaging, and grouping and administration was started. Principle of grouping: mean values of tumor chemiluminescence for mice in all groups are equal. The dosing regimen is the same as in Figure 15. (Figure 16A) Representative photos of mice lung metastasis on day 31 after administration. The lungs of each group of mice were taken out and soaked in picric acid-formaldehyde solution overnight, and the naked-eye visible lung metastatic foci were counted. The black arrows indicate the tumor metastatic foci. (Figure 16B) Quantitative data of the metastasis to lung, liver and spleen of mice on day 31 after dosing. The lungs, livers and spleens of the mice were taken out, and the naked-eye visible metastatic foci were counted. (Figure 16C) Survival curves of mice in each group after administration. TH-Z 93 + PD-1 survival rate, CDI = 0.9, Sim + PD-1 survival rate, CDI = 0.78. (Figure 16D) Change in body weight of mice during the administration. There were no significant differences between the groups. **: P <0.01, ***: P <0.001, ns: No significant difference.

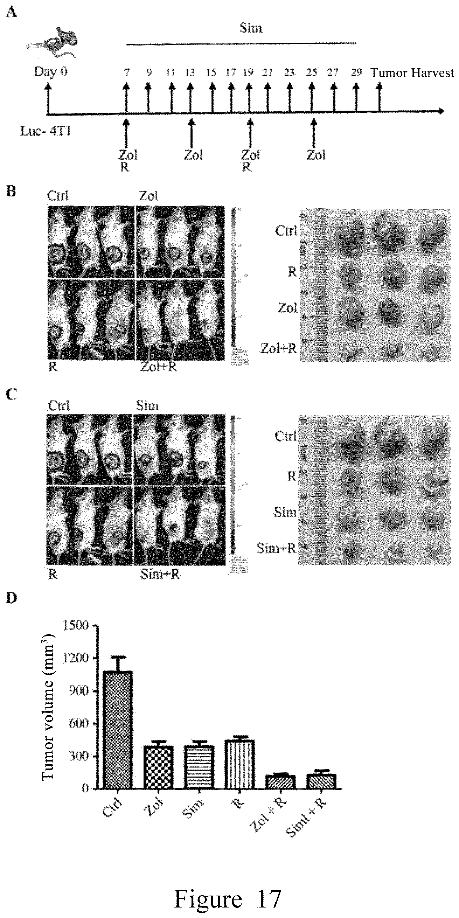

Figure 17 shows the combined antitumor effect of Sim, and Zol with the radiation therapy. 1x10⁶ 4T1 cells were inoculated subcutaneously in the left hip of 6-week-old female BALB/c mice. The tumor volume was measured using a vernier caliper, and the tumor volume was calculated using the formula of length (mm) × width² (mm)/2. Grouping and administration and irradiation treatment were started on day 7 after tumor growth. Dosing or irradiation regimen: Zol administration group, 1 mg/kg, once every 6 days for a total of 4 times; Sim administration group, 2 mg/kg, once every two days for a total of 12 times; the irradiation was performed at a dose of 6 Gy/mouse, on the 7th and 19th days after tumor transplantation. The tumor of the mice was exposed to irradiation only. On the 31st day, mice were injected with potassium luciferin for in-vivo imaging, and the tumors of the mice were taken out for photo recording. (Figure 17A) Experimental flowchart. (Figure 17B) In-vivo imaging of mice (left) and tumor size images (right) treated by Zol combined with the radiation therapy. (Figure 17C) In-vivo imaging of mice (left) and tumor size images (right) treated by Sim combined with the radiation therapy. (Figure 17D) Tumor volume at the end of the experiment after Zol and Sim combined with the radiation therapy (day 31 after the tumor transplantation). Zol + R, CDI = 0.73; Sim + R, CDI = 0.79.

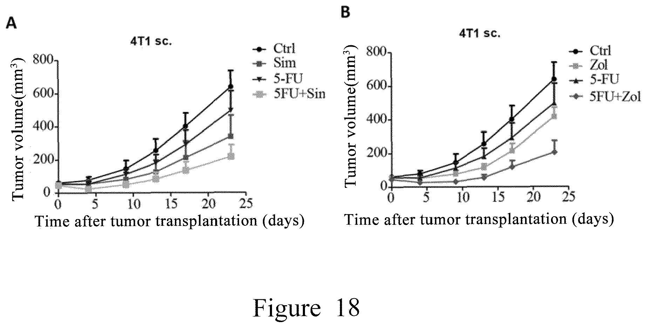

Figure 18 shows the inhibition effect of Sim, and Zol in combination with 5-FU on the tumor growth. 1x10⁶ 4T1 cells were seeded subcutaneously in the left side of 6-week-old female BALB/c mice. Zol and Sim were administered intraperitoneally (i.p.). The tumor volume was measured using a vernier caliper. Grouping and administration and 5-FU treatment were started on the 9th day after tumor transplantation. The experiment included 6 groups. Dosing regimen: control group; Sim group; Zol group; 5-FU group; Sim + 5-FU group; Zol + 5-FU group. Dosing regimen: Zol administration group, dissolved in sterile PBS, 1 mg/kg, once every 6 days for a total of 3 times; Sim administration group, dissolved in 75% DMSO, 2 mg/kg, once every two days for a total of 7 times. 5-FU, 50 mg/kg, administered once in total (Zol or Sim was injected 24 hours after 5-FU administration). (Figure 18A) Sim + 5-FU, CDI = 0.82; (Figure 18B) Zol + 5-FU, CDI = 0.79.

### DETAILED DESCRIPTION

In particular, the present disclosure relates to the following embodiments.

### Mevalonate pathway inhibitor

Mevalonate pathway inhibitors specifically include the following types: 1) Thiolase (acetoacetyl-CoA transferase) inhibitors; 2) HMG-CoA synthase inhibitors; 3) HMG-CoA reductase inhibitors, such as statins; 4) Mevalonate kinase inhibitors; 5) phosphonomevalonate kinaseinhibitors; 6) Mevalonate-5-pyrophosphate decarboxylase inhibitors; 7) isopentenyl-diphosphate isomerase Inhibitors; 8) Farnesyl diphosphate synthase inhibitors, such as bisphosphonate drugs; 9) Geranylgeranyl diphosphate synthase inhibitors; and 10) Geranylgeranyl transferase (I, II) inhibitors.

The thiolase (acetoacetyl-CoA transferase) inhibitor includes, but is not limited to, L-660631 described in biochemical and biophysical research communications, 1989, 163, 548-553.

The HMG-CoA synthase inhibitor includes, but is not limited to, L-659699 described in Biochem. J (1993) 289, 889-895, 1233A/F-244 described in Agric. Biol. Chem., 55 (12), 3129-3131, 1991, as well as Dihydroxerulin described in Tetrahedron, 2000, 56(3), 479-487 and those compounds disclosed in the following documents: US5064856; EP0411703A1; Agric. Biol. Chern., 1991, 55 (12):3129-3131; Bioorg. Med. Chem., 1998, 6:1255-1272; Biochem. Biophys. Res. Commun., 1999, 265:536-540. All of these patents and other publications are incorporated herein by reference.

The HMG-CoA reductase inhibitor includes, but is not limited to, those compounds disclosed in the following documents: US5102911-A; EP476493-A1; US5091378-A; EP465970-A; EP465265-A; EP464845-A; EP463456-A; EP456214-A1; EP591165-A; US5049577-A; EP445827-A2; EP442495-A; US5025000-A; EP435322-A2; US5023250-A; JP3112967-A; US5017716-A; US5010105-A; US5011947-A; EP424929-A1; EP422895-A1; EP420266-A2; EP419856-A2; EP418648-A1; EP416383-A2; US4996234-A; US4994494-A; EP415488-A; US4992429-A; EP411420-A2; EP409399-A1; EP408806-A1; DE3918364-A; EP401705-A; EP391185-A1; US4957940-A; US4950675-A; US4946860-A; US4940727-A; US4939143-A; US4937264-A; US4937263-A; EP402154-A1; EP375156-A2; US4929620-A; US4927851-A; US4904692-A; EP468974-A1; US4904646-A; WO9113616-A1; US4897402-A; US4892884-A; EP355846-A2; US4885314-A; EP349063-A; US4876280-A; EP346759-A2; EP422102-A1; EP344602-A1; DE3805884-A; EP330172-A; EP327166-A; EP327165-A; WO8905639-A; JP1068367-A; EP306264-A; US4792614-A; DE3632893-A1; US4719229-A; EP251625-A2; EP232997-A1; EP211416-A2; EP183132-A2; EP164049-A; FR2516087-A1; Wang K et al. J. Nat. Prod., 2015, 78: 1977-1989; Wess G et al. J Med. Chem., 1994, 37: 3240-3246; Procopiou PA et al. J Med. Chem., 1993, 36: 3655-3662; Pfefferkorn JA et al. J Med. Chem., 2008, 51: 31-45; Ahmad S et al. J Med. Chem., 2008, 51: 2722-2733; Sarver RW et al. J Med. Chem., 2008, 51: 3804-3813 all of these patents and other publications are incorporated herein by reference.

In an alternative embodiment, the HMG-CoA reductase inhibitor is a statin compound. Exemplary statin compounds are selected from the group consisting of: pravastatin, atorvastatin, rosuvastatin, fluvastatin, pitavastatin, mevastatin, lovastatin, simvastatin, cerivastatin, or a pharmaceutically acceptable salt, ester, prodrug, or solvate thereof. Preferred HMG-CoA reductase inhibitors are those that have been marketed, most preferably simvastatin, lovastatin and mevastatin, or a pharmaceutically acceptable salt, ester, prodrug and solvate thereof. Methods for preparing HMG-CoA reductase inhibitors are well known to those skilled in the art and include those are commercially available. A HMG-CoA reductase inhibitor may be used in its free acid form, its ester form, or a pharmaceutically acceptable salt thereof. These pharmaceutically acceptable salts include, for example, sodium salts, calcium salts, aluminum salts and ester salts. A HMG-CoA reductase inhibitor can be used in the form of racemic mixtures, or more active, appropriate stereoisomers.

The farnesyl diphosphate synthase inhibitors include, but are not limited to, those compounds disclosed in the following documents: US7462733; US20080200679; WO2006039721; US7358361; US7745422; US20100316676; WO2007109585; US7687482; WO2008128056; US20080255070; WO2010033980; WO2010033981; WO2008076417; US7781418; WO2010033978; WO2009068567; WO2010043584; WO2009128918; ACS Med. Chem. Lett. 2013, 4:423-427; J. Am. Chem. Soc., 2009, 131:5153-62; Nat. Chem. Biol., 2010, 6:660-6; Bioorg. Med. Chem. Lett., 2008, 18:2878-82; J. Med. Chem., 2008, 51:2187-95; Proc. Natl. Acad. Sci. U. S. A., 2007, 104:10022-7; Tetrahedron Lett. 2011, 52:2285-87; Chem. Commun., 2010, 46:5340-5342; Expert Opin. Ther. Pat. 2011, 21(9): 1433-1451; J. Pharmacol. Exp. Ther., 2001, 296:235-42; J. Med. Chem. 2003, 46:5171-5183; J. Med. Chem. 2005, 48:2957-2963; J. Med. Chem. 2006, 49:5804-5814; J. Med. Chem. 2013, 56:7939-7950; J. Med. Chem. 2008, 51:2187-2195; ChemMed Chem 2015, 10:1884-1891; Biochim. Biophys. Acta, 2014, 1840 1840:1051-1062; J. Med. Chem. 2007; 50:5967-75, all of these patents and other publications are incorporated herein by reference.

In a further embodiment, the farnesyl diphosphate synthase inhibitor is a bisphosphonate compound or a pharmaceutically acceptable salt, ester, prodrug, or solvate thereof. The term "bisphosphonic acid (phosphonate)" refers to a compound characterized by two phosphonate groups linked by phosphoether bonds to a central (geminal) carbon atom. This P-C-P structure is shown in Formula I below. It should be noted that the term "bisphosphonic acid (phosphonate)" as used herein referring to the therapeutic agents of the present disclosure are meant to also encompass bisphosphonates, biphosphonic acids, and salts and derivatives thereof. Unless specifically indicated, the use of a specific nomenclature in referring to the biphosphonic acid or bisphosphonate is not meant to limit the scope of the present disclosure.

Bisphosphonates as pharmaceutical agents are described for example in EP-A-170,228, EP-A-197,478, EP-A-22,751, EP-A-252,504, EP-A-252,505, EP-A-258,618, EP-A-350,002, EP-A-273,190 and WO-A-90/00798, and the like, all of which are incorporated herein by reference.

"Bisphosphonatess and pharmaceutically acceptable salts thereof' as pharmaceutical agents are described for example in U.S. Patent Nos. 4,509,612, 4,666,895, 4,719,203, 4,777,163, 5,002,937, 4,971,958 and 4,958,839 and European Patent Applications 252,504 and 252,505, all of which are incorporated herein by reference.

Preferred bisphosphonates or pharmaceutically acceptable salts thereof are selected from the group consisting of alendronic acid, simma phosphonic acid, clodronic acid, EB-1053, tiludronic acid, etidronic acid, ibandronic acid, incadronic acid, minodronic acid, neridronic acid, olpadronic acid, risedronic acid, piridronic acid, pamidronic acid, zoledronic acid or an acceptable salt thereof, such as ibandronic acid monosodium salt monohydrate.

The geranylgeranyl diphosphate synthase inhibitor includes, but is not limited to, those compounds disclosed in the following documents: J. Med. Chem. 2009, 52:8025-37; Biochem. Biophys. Res. Commun., 2007, 353:921-925; J. Med. Chem. 2002, 45:2185-2196; Bioorg. Med. Chem. 2008, 16:390-399; J. Med. Chem., 2008, 51:5594-5607; ACS Med. Chem. Lett. 2015, 6:1195-1198; Proc. Natl. Acad. Sci. U. S. A., 2012, 109(11):4058-4063, all of which are incorporated herein by reference.

The geranylgeranyl transferase (I, II) inhibitor includes, but is not limited to, those compounds disclosed in the following documents: EP1165084A1; EP1165084A4; EP2014291A2; EP2014291B1; US6103487; US6284910; US6355643B1; US6586461B1; US6638962B2; US7763620B2; US8093274B2; US8815935B2; US9040563B2; US20030219847A1; US20040121985A1; US20060030624A1; US20070249010A1; US20100063114A1; US20110178138A1; US20120035184A1; US20130102639A1; WO1999006376A1; WO2000033826A1; WO2000051614A1; WO2007111948A2; WO2007118009A1; WO2010014054A1; WO2010088457A2; WO2012034038A2; WO2009106586; Angew. Chem. Int. Ed. 2011, 50, 4957-4961; J. Med. Chem. 2010, 53:3454-64; J. Biol. Chem. 2001, 276:48213-22; Bone. 2005, 37:349-58; J. Biol. Chem. 2009, 284:6861-8; Eur. J. Med. Chem., 2011, 46(10):4820-4826; Drug Discov. Today, 2015, 20:267-276; J. Med. Chem. 2012, 55, 8330-8340; J. Am. Chem. Soc. 2007, 129:5843-5845; J. Med. Chem. 2009, 52:8025-8037; J. Med. Chem. 1999, 42:1333-1340; PLoS ONE, 2011, 6:e26135; Bioorg. Med. Chem., 2005, 13:677-688; IL Farmaco, 2004, 59:857-861; Org. Biomol. Chem., 2006, 4, 1768-1784; J. Biol. Chem., 2006, 281(18):12445-12450; J. Biol. Chem., 2008, 283(15):9571-9579; and GGTI-298, which is described in McGuire et al (1996) Platelet-derived growth factor receptor tyrosine kinase phosphorylation requires protein geranylgeranylation but not farnesylation. J. Biol. Chem. 271 27402. PMID: 8910319, and has the following structure: 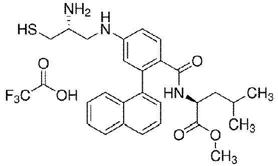 The structure of GGTI-298 all of which are incorporated herein by reference.
[1] In one aspect, the mevalonate pathway inhibitor used in the present disclosure is a HMG-CoA reductase inhibitor, preferably a statin. In an alternative embodiment, the statin is selected from pravastatin, atorvastatin, rosuvastatin, fluvastatin, pitavastatin, mevastatin, lovastatin, simvastatin, or cerivastatin, or their pharmaceutically acceptable salts, esters, prodrugs, or solvates. In another alternative embodiment, the statin is selected from simvastatin, lovastatin, or mevastatin, or a pharmaceutically acceptable salt, ester, prodrug, or solvate thereof. In another alternative embodiment, the statin is simvastatin or a pharmaceutically acceptable salt, ester, prodrug, or solvate thereof.
[2] In another aspect, the mevalonate pathway inhibitor used in the present disclosure is a farnesyl diphosphate synthase inhibitor. In an alternative embodiment, the farnesyl diphosphate synthase inhibitor is a bisphosphonate compound or a pharmaceutically acceptable salt, ester, prodrug, or solvate thereof. In another alternative embodiment, the bisphosphonate compound is selected from the group consisting of zoledronic acid, pamidronic acid, alendronic acid, ibandronic acid, neridronic acid, risedronic acid, olpadronic acid, or minodronic acid, or a pharmaceutically acceptable salt, ester, prodrug, or solvate thereof. In another alternative embodiment, the bisphosphonates compound is zoledronic acid, or a pharmaceutically acceptable salt, ester, prodrug, or solvate thereof.

In another alternative embodiment, the farnesyl diphosphate synthase inhibitor is a compound of formula I or a pharmaceutically acceptable salt, ester, prodrug, or solvate thereof: 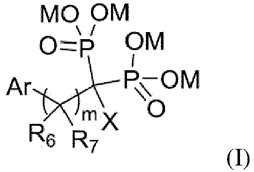 in the formula I, the compound has a molecular weight of less than 1000, and Ar is a benzimidazolyl-type group, or an aza-benzimidazolyl group;
X is selected from the group consisting of hydrogen, hydroxy, aliphatic group, mercapto, halogen, alkoxy and alkyl; each M is independently selected from the group consisting of negative charge, hydrogen, alkyl, aliphatic group, -(CH₂)ₚ-O-CO-R, -(CH₂)ₚ-CO-R and positive ion; wherein p is an integer of 1 to 6, R is hydrogen, alkyl or aryl; the positive ion is Li⁺, Na⁺, K⁺, Ca²⁺, Mg²⁺, NH₄⁺ or N(R')₄⁺, wherein R' is alkyl; R₆ and R₇ are each independently selected from the group consisting of hydrogen, hydroxy, mercapto, halogen, amino, aliphatic group and alkyl; m is an integer of 1 to 6.

In another alternative embodiment, the compound represented by formula I is a compound represented by the following formula II-X: 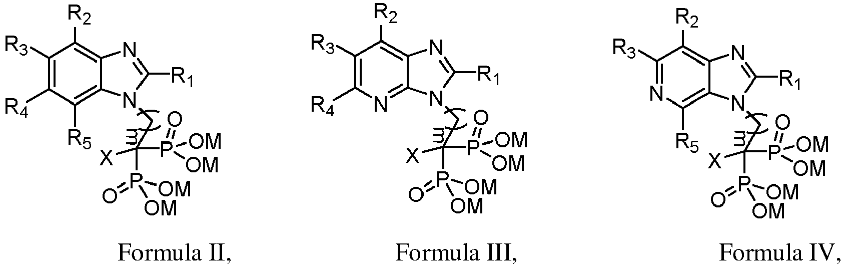 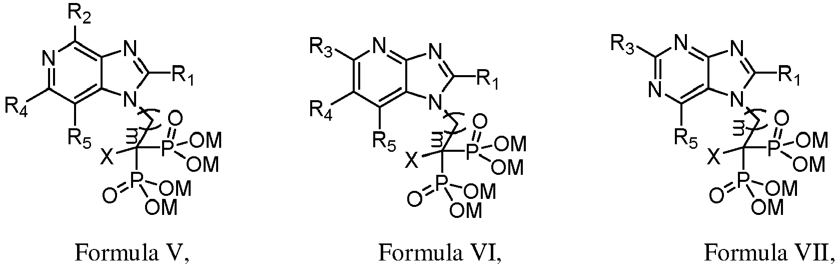 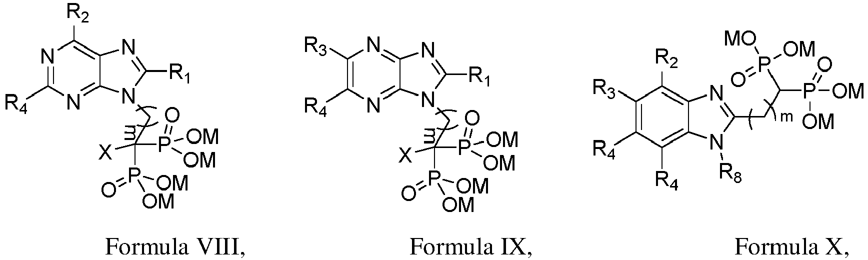 in the Formulae II-X, X is selected from the group consisting of hydrogen, hydroxy, mercapto, halogen, alkoxy and alkyl;
each M is independently selected from the group consisting of negative charge, hydrogen, alkyl, -(CH₂)ₚ-O-CO-R, -(CH₂)ₚ-CO-R and positive ion; wherein p is an integer of 1 to 6, R is hydrogen, alkyl or aryl; the positive ion is Li⁺, Na⁺, K⁺, Ca²⁺, Mg²⁺, NH₄⁺ or N(R')₄⁺, wherein R' is alkyl;
R₁, R₂, R₃, R₄, R₅ and R₈ are independently selected from the group consisting of hydrogen, hydroxy, aliphatic group, mercapto, halogen, amino, alkyl, -O-(CH₂)_{q}CH₃, -NH-(CH₂)_{q}CH₃, -N[(CH₂)_{q}CH₃]₂, -(CH₂)ₚ-S-(CH₂)_{q}CH₃, -O-(CH₂)ₚ-S-(CH₂)_{q}CH₃, and -O-(CH₂)ₚ-O-(CH₂)_{q}CH₃, wherein p is an integer of 1 to 6, q is an integer of 0 to 6; m is an integer of 1 to 6.

In another alternative embodiment, wherein the compound is a compound represented by formula XI-XVIII: 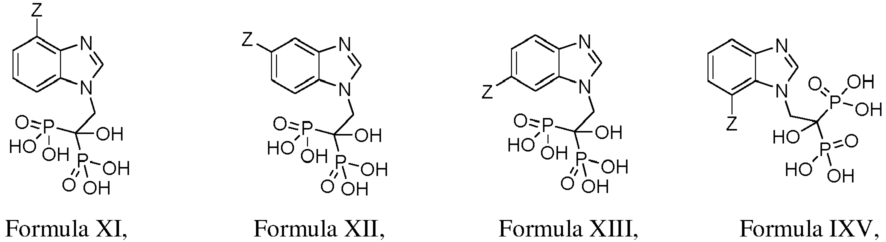 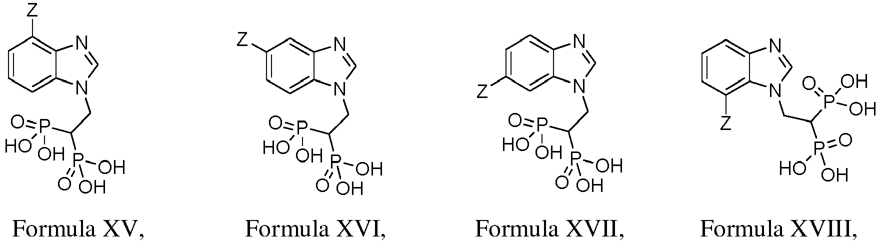 in the Formulae XI-XVIII, Z is hydrogen, hydroxy, aliphatic group, alkoxy, amino or alkylamino.

In another alternative embodiment, wherein the compound is a compound represented by formula IXX or XX: 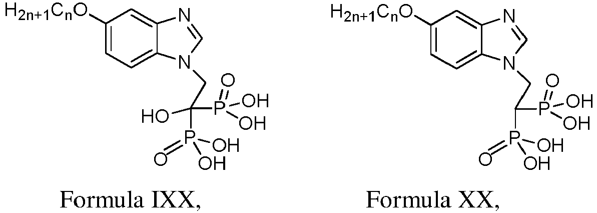 in Formulae IXX and XX, n is an integer of 0, or 1-12.

In another alternative embodiment, the compound is selected from the group consisting of

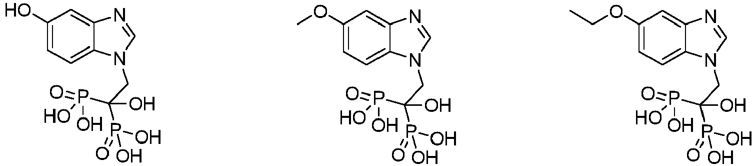

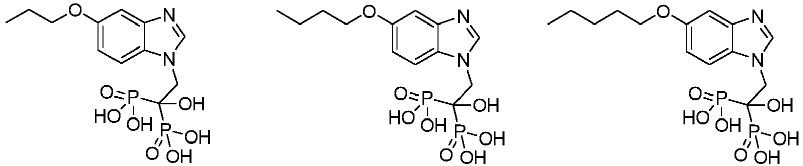

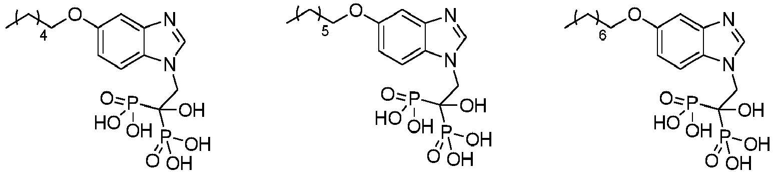

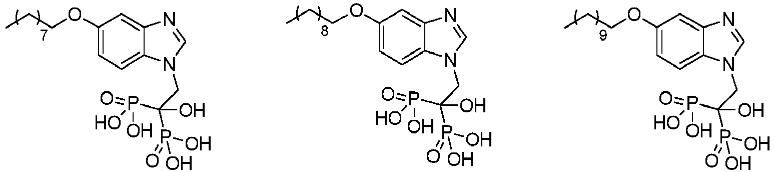

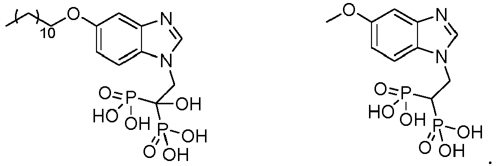

In another alternative embodiment, wherein said compound of formula I is a compound of the following formula or a pharmaceutically acceptable salt, ester, prodrug, or solvate thereof: 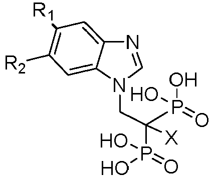 wherein R₁ and R₂ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl; X is selected from the group consisting of hydrogen, hydroxy, mercapto, and halogen.

In another alternative embodiment, wherein said compound of formula I is a compound of the following formula or a pharmaceutically acceptable salt, ester, prodrug, or solvate thereof: 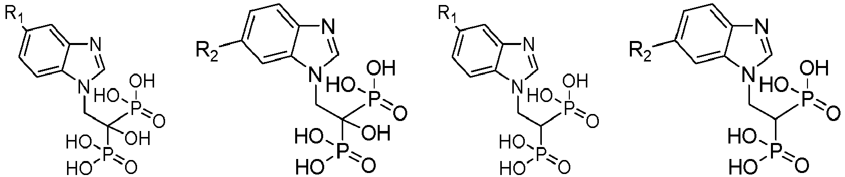 wherein R₁ and R₂ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl.

In another alternative embodiment, wherein said compound of formula I is a compound of the following formula or a pharmaceutically acceptable salt, ester, prodrug, or solvate thereof: 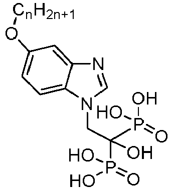 where n is an integer of 1-24. Preferably, wherein n is an integer of 1-20. Preferably, wherein n is an integer of 1-15. Preferably, wherein n is an integer of 1-12. Preferably, wherein said compound is selected from:

| n= | Compound No. |
|---|---|
| 1 | TH-Z79 |
| 2 | TH-Z148 |
| 3 | TH-Z149 |
| 4 | TH-Z150 |
| 5 | TH-Z151 |
| 6 | TH-Z80 |
| 7 | TH-Z152 |
| 8 | TH-Z81 |
| 9 | TH-Z153 |
| 10 | TH-Z82 |
| 11 | TH-Z154 and |
| 12 | TH-Z155. |

In another alternative embodiment, wherein said compound of formula I is a compound of the following formula or a pharmaceutically acceptable salt, ester, prodrug, or solvate thereof: 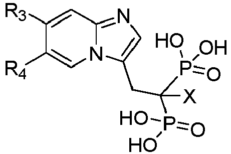 wherein R₃ and R₄ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl; X is selected from the group consisting of hydrogen, hydroxy, mercapto, and halogen.

In another alternative embodiment, wherein said compound of formula I is a compound of the following formula or a pharmaceutically acceptable salt, ester, prodrug, or solvate thereof: 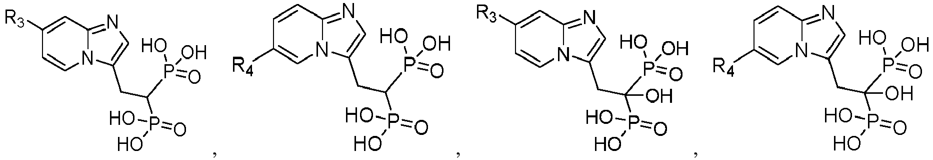 wherein R₃ and R₄ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl.

In another alternative embodiment, wherein said compound of formula I is a compound of the following formula or a pharmaceutically acceptable salt, ester, prodrug, or solvate thereof: 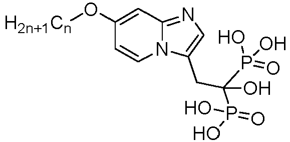 wherein n is an integer of 1-24. Preferably, wherein n is an integer of 1-20. Preferably, wherein n is an integer of 1-15. Preferably, wherein n is an integer of 1-12. Preferably, wherein said compound is selected from:

| n= | Compound No. |
|---|---|
| 1 | TH-Z156 |
| 2 | TH-Z157 |
| 3 | TH-Z158 |
| 4 | TH-Z159 |
| 5 | TH-Z160 |
| 6 | TH-Z97 |
| 7 | TH-Z161 |
| 8 | TH-Z98 |
| 9 | TH-Z162 |
| 10 | TH-Z99 |
| 11 | TH-Z198 and |
| 12 | TH-Z163. |

Preferably, wherein the compound of formula I is a compound of the following formula or a pharmaceutically acceptable salt, ester, prodrug, or solvate thereof: 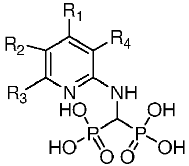 wherein
R₁ is selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl;
R₂ is selected from the group consisting of hydrogen, alkyl, alkoxy, alkylamino, alkylthio, halogen, hydroxy, cycloalkyl, heterocyclyl, aryl and heteroaryl;
R₃ is selected from the group consisting of hydrogen, alkyl, alkoxy, alkylamino, alkylthio, halogen, hydroxy, cycloalkyl, heterocyclyl, aryl and heteroaryl;
or R₂ and R₃ together with the carbon atom to which they are attached form an aromatic or heteroaromatic ring; and
R₄ is selected from the group consisting of hydrogen, alkyl, alkoxy, alkylamino, alkylthio, halogen, hydroxy, cycloalkyl, heterocyclyl, aryl and heteroaryl.

Preferably, R₁ is selected from the group consisting of hydrogen, C₁₋₁₀ alkyl, C₁₋₁₀ alkynyl, C₁₋₁₀ alkylamino, C₁₋₁₀ alkylthio, halogen, hydroxy, indazolyl, C₁₋₁₀ alkoxy, and C₁₋₁₀ alkoxy substituted with phenyl or pyridyl, wherein the pyridyl is optionally substituted with carbamoyl. Preferably, R₁ is selected from the group consisting of hydrogen, 4-methylphenylethoxy, 4,5,6,7-tetrahydro-2H-indazol-2-yl, (2-carbamoylpyridin-4-yl)methoxy, benzyloxy, hexyloxy, methylthio, octylamino, hexyl, octyl, decyl, oct-1-yn-1-yl, hydroxyl, and bromo.

R₂ is selected from the group consisting of hydrogen, C₁₋₁₀ alkoxy, and halogen. Preferably, R₂ is selected from the group consisting of hydrogen, octyloxy, and bromo.

R₃ is selected from the group consisting of hydrogen, C₁₋₁₀ alkyl, and C₁₋₁₀ alkoxy. Preferably, R₃ is selected from the group consisting of hydrogen, methyl, and hexyloxy.

Alternatively, R₂ and R₃ together with the carbon atom to which they are attached form a benzene ring.

R₄ is selected from the group consisting of hydrogen, and C₁₋₁₀ alkoxy. Preferably, R₄ is selected from the group consisting of hydrogen and octyloxy.

Preferably, wherein said compound is selected from:

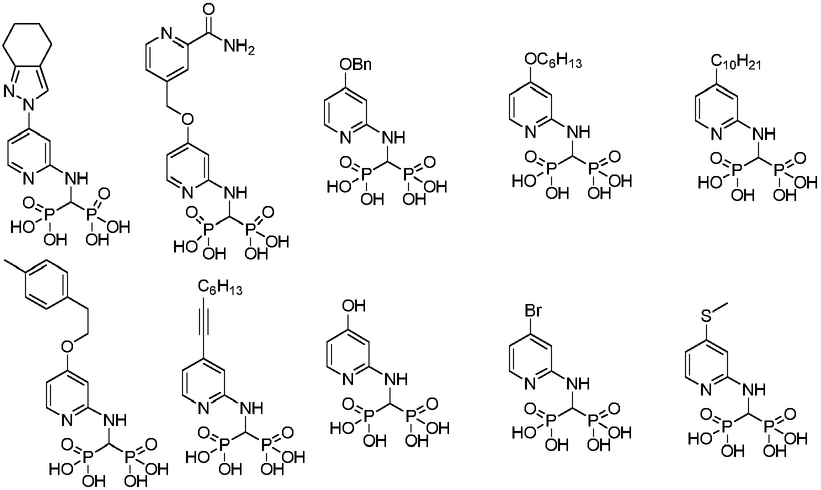

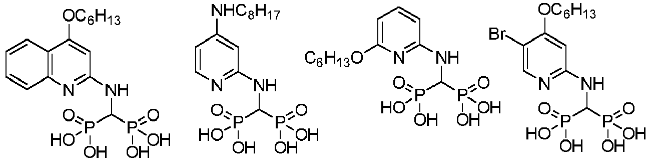

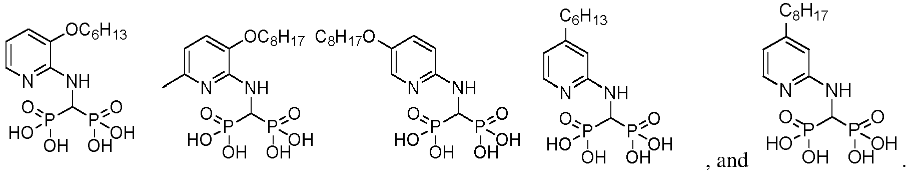

Preferably, wherein the compound of formula I is a compound of the following formula or a pharmaceutically acceptable salt, ester, prodrug, or solvate thereof: 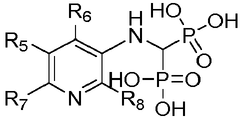 wherein:
R₅ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₆ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₇ is selected from the group consisting of hydrogen, alkyl, alkenyl, alkynyl, alkoxy, alkylamino, alkylthio, halogen, hydroxy, cycloalkyl, heterocyclyl, aryl and heteroaryl; and
R₈ is selected from the group consisting of hydrogen, alkyl, alkenyl, alkynyl, alkoxy, alkylamino, alkylthio, halogen, hydroxy, cycloalkyl, heterocyclyl, aryl and heteroaryl.

Preferably, R₅ is C₁₋₁₀ alkoxy.

Preferably, wherein the compound is

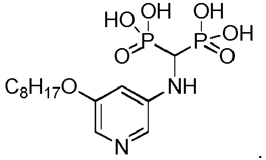

[3] In another aspect, the mevalonate pathway inhibitor used in the present disclosure is a geranylgeranyl diphosphate synthase inhibitor.

In an alternative embodiment, the geranylgeranyl diphosphate synthase inhibitor is a compound of the following formula or a pharmaceutically acceptable salt, ester, prodrug, or solvate thereof: 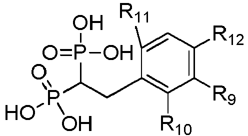 wherein
R₉ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₁₀ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₁₁ is selected from the group consisting of hydrogen, alkyl, alkenyl, alkynyl, alkoxy, alkylamino, alkylthio, halogen, hydroxy, cycloalkyl, heterocyclyl, aryl and heteroaryl; and
R₁₂ is selected from the group consisting of hydrogen, alkyl, alkenyl, alkynyl, alkoxy, alkylamino, alkylthio, halogen, hydroxy, cycloalkyl, heterocyclyl, aryl and heteroaryl.

Preferably, R₉ is C₁₋₁₀ alkoxy.

Preferably, wherein the compound is

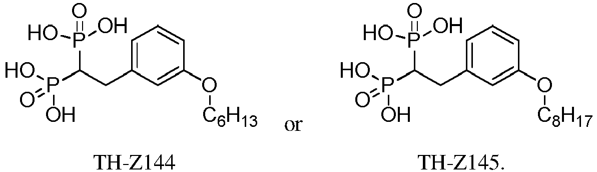

### Immune checkpoint inhibitor

As early as in the 1980s, French scientists discovered a molecule, cytotoxic T-lymphocyte antigen 4, CTLA-4, on the surface of T cells. Under normal circumstances, CTLA-4 is expressed on the plasma membrane of activated T cells. By inhibiting the binding of T-cell co-stimulatory molecule CD28 to its ligand B7 and inhibiting the cell cycle of T cell, it negatively regulates T cell function (Reversal of the TCR stop signal by CTLA-4. Science (2006) 313:1972-75; Modulation of TCR-induced transcriptional profiles by ligation of CD28, ICOS, and CTLA-4 receptors. Proc. Natl. Acad. Sci. U. S. A. (2002) 99:11790-95). So, immunologist Allison et al speculated that inhibiting the CTLA-4 molecule can promote T cell function and destroy tumors. In 1996, Allison et al. firstly published the CTLA-4 antibody to treat tumors in Science (Enhancement of antitumor immunity by CTLA-4 blockade. Science (1996) 271:1734-36). Subsequently, a humanized CTLA-4 antibody was developed by a biological company named Medarex, which was acquired by Bristol-Myers Squibb (BMS). In 2011, the humanized CTLA-4 antibody was marketed with the name Ipilimumab. Ipilimumab has been tested on more than 1,800 patients before marketing approval, wherein the patient survival rate is approximately 20% after at least 3 years of treatment (Four-year survival rates for patients with metastatic melanoma who received Ipilimumab in phase II clinical trials. Ann. Oncol. (2013) 24:2174-80). As a result, Ipilimumab is the first medicine which can significantly prolong bioantibodies in patients with advanced melanoma and the first immune checkpoint inhibitor. In addition to its use in patients with advanced melanoma, Ipilimumab is also used in combination with carboplatin and paclitaxel in the treatment of patients with non-small cell lung cancer, and it has a longer progression-free survival than monotherapy (Ipilimumab in Combination With Paclitaxel and Carboplatin As First-Line Treatment in Stage IIIB/IV Non-Small-Cell Lung Cancer: Results From a Randomized, Double-Blind, Multicenter Phase II Study. J. Clin. Oncol. (2012) 30:2046-54).

In addition, there is a more successful antibody molecule than the CTLA-4 antibody. In the 1990s, Japanese biologists discovered that a molecule is expressed on the surface of dying T cells and named the molecule as programmed death 1, PD-1. The binding of PD-1 to its ligands PDL-1 and PDL-2 leads to the programmed death of effector T-cells (Tumor-associated B7-H1 promotes T-cell apoptosis: A potential mechanism of immune evasion. Nat. Med. (2002) 8:793-800). At the same time, other immune cells, such as B cells and natural killer cells, also express PD-1. Therefore, the inhibition of PD-1 may have other immune effects besides T cells. At present, a variety of antibodies against PD-1/PD-L1/PD-L2 targets have entered clinical research (Table 1). In phase I clinical trials, Nivolumab and Pembrolizumab have lower toxicity and good therapeutic effects in patients with various tumors such as advanced melanoma, non-small cell lung cancer, renal cell carcinoma and in other solid tumor patients (Sharfman WH, et al. Survival, Durable Tumor Remission, and Long-Term Safety in Patients With Advanced Melanoma Receiving Nivolumab. J. Clin. Oncol. (2014) 32:1020-30). In a phase III melanoma clinical trial, wherein patients were treated with Ipilimumab but the disease was still progressing, after Nivolumab treatment, 32% of patients had complete remission, compared with the 11% complete remission rate of the carbamazine group or carboplatin/paclitaxel combination group. PD-1 antibody showed significant efficacy and low side effects. Similarly, Pembrolizumab has been approved by the US Food and Drug Administration for patients with melanoma who have failed Ipilimumab treatment. Compared with the solid tumor efficacy of Nivolumab and Pembrolizumab, Pidilizumab alone or combined with rituximab is more commonly used in the treatment of hematologic malignancies. In addition, PD-L1 antibodies such as BMS-956559, MPDL3280A, MEDI4736, MSB0010718C have significant therapeutic effects in the early treatment of various cancers such as bladder cancer, head and neck cancer, and gastrointestinal malignancies (Preliminary data from a multi-arm expansion study of MEDI4736, an anti-PD-L1 antibody. J. Clin. Oncol. (2014) 32; Phase I open-label, multiple ascending dose trial of MSB0010718C, an anti-PD-Ll monoclonal antibody, in advanced solid malignancies. J. Clin. Oncol. (2014) 32).

**Table 1 Antibodies targeting PD-1/PD-L1/PD-L2 have entered clinical studies.**

| Target | Antiboby name | Production Company |
|---|---|---|
| PD-1 | Nivolumab | Bristol-Myers Squibb |
| | Pembrolizumab | Merck |
| | Pidilizumab | CureTech |
| PD-L1 | MPDL3280A | Genentech |
| | MEDI4736 | MedImmune/AstraZeneca |
| | BMS-936559 | Bristol-Myers Squibb |
| | MSB0010718C | EMD Serono |
| PD-L2 fusion protein | AMP-224 | Amplimmune |
| | Nivolumab | Bristol-Myers Squibb |

### Any drug capable of causing neo-antigen production

Any drug capable of causing neo-antigen production referred to in the present disclosure is selected from 5-FU, 5-azacytidine, 5-aza-2'-deoxycytidine, thiopurine, thioguanine, nelarabine, cladribine, clofarabine, capecitabine, cytarabine, ancitabine, gemcitabine, troxacitabine, decitabine; doxorubicin, mitoxantrone, daunorubicin, epirubicin and idarubicin; oxaliplatin, enloplatin, carboplatin, nedaplatin, dicycenloplatin, cisplatin; bortezomib; and cetuximab, preferably, any drug capable of causing neo-antigen production is 5-FU, azacytidine, deoxycytidine, doxorubicin, mitoxantrone, oxaliplatin, bortezomib, and cetuximab.

### Definition

### Term

To facilitate review of various embodiments in the present disclosure, the following explanations of terms are provided. Other terms and explanations are provided in the context of this disclosure.

Unless otherwise explained, all technical and scientific terms used herein have the same meaning as commonly understood by one of ordinary skill in the art to which this disclosure pertains.

The "immune response" is a response of a cell of the immune system, such as a B cell, T cell, or monocyte, to a stimulus. An immune response can be a B cell response, which results in the production of specific antibodies, such as antigen specific neutralizing antibodies. An immune response can also be a T cell response, such as a CD4+ response or a CD8+ response. In some cases, the response is specific for a particular antigen (that is, an "antigen-specific response"). If the antigen is derived from a pathogen, the antigen-specific response is a "pathogen-specific response." A "protective immune response" is an immune response that inhibits a detrimental function or activity of a pathogen, reduces infection by a pathogen, or decreases symptoms (including death) that result from infection by the pathogen. A protective immune response can be measured, for example, by the inhibition of viral replication or plaque formation in a plaque reduction assay or ELISA-neutralization assay, or by measuring resistance to pathogen challenge *in vivo.*

The term "antibody" describes an immunoglobulin whether natural or partly or wholly synthetically produced. The term also covers any polypeptide or protein having a binding domain which is, or is homologous to, an antigen-binding domain.

A tumor antigen is an antigenic substance that is produced in a tumor cell, i.e. it may trigger an immune response in a host. A tumor antigen is a tumor marker useful in diagnostic tests for identifying tumor cells and are potential candidates for use in cancer therapy.

As used herein, the term "optionally" means that the subsequently described event(s) may or may not occur, and includes both event(s) that occur and event(s) that do not occur.

The term "substituted," as used herein, means that any one or more hydrogens on the designated atom or ring is replaced with a selection from the indicated group, provided that the designated atom's or ring atom's normal valency is not exceeded, and that the substitution results in a stable compound. When a substituent is keto (i.e., =O), then 2 hydrogens on the atom are replaced.

When enumerating the range of values, it is intended to include each of the values and sub-ranges within the range. For example, "C₁₋₆ alkyl" is intended to include C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

As used herein, "aliphatic" or "aliphatic group" includes alkyl, alkenyl and alkynyl groups as defined below.

As used herein, "alkyl" is intended to include both branched and straight-chain saturated aliphatic hydrocarbon groups containing 1 to 20 carbons, preferably 1 to 12 carbons, preferably 1 to 11 carbons, preferably 1 to 10 carbons, preferably 1 to 9 carbons, more preferably 1 to 8 carbons, in the chain, such as methyl, ethyl, propyl, isopropyl, butyl, *t*-butyl, isobutyl, pentyl, hexyl, isohexyl, heptyl, 4,4-dimethylpentyl, octyl, 2,2,4-trimethyl-pentyl, nonyl, decyl, undecyl, dodecyl, the various branched chain isomers thereof, and the like as well as such groups may optionally include 1 to 4 substituents such as halo, for example F, Br, Cl, or I, or CF₃, alkyl, alkoxy, aryl, aryloxy, aryl(aryl) or diaryl, arylalkyl, arylalkyloxy, alkenyl, cycloalkyl, cycloalkylalkyl, cycloalkylalkyloxy, amino, hydroxy, hydroxyalkyl, acyl, heteroaryl, heteroaryloxy, heteroarylalkyl, heteroarylalkoxy, aryloxyalkyl, alkylthio, arylalkylthio, aryloxyaryl, alkylamido, alkanoylamino, arylcarbonylamino, nitro, cyano, thiol, haloalkyl, trihaloalkyl, and/or alkylthio.

Unless otherwise indicated, the term "alkenyl" as used herein by itself or as part of another group refers to straight or branched chain radicals of 2 to 20 carbons, preferably 2 to 12 carbons, preferably 2 to 11 carbons, preferably 2 to 10 carbons, preferably 2 to 9 carbons, and more preferably 1 to 8 carbons in the chain, which include one to six double bonds in the normal chain, such as vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl, 4,8,12-tetradecatrienyl, and the like, and which may be optionally substituted with 1 to 4 substituents, namely, halogen, haloalkyl, alkyl, alkoxy, alkenyl, alkynyl, aryl, arylalkyl, cycloalkyl, amino, hydroxy, heteroaryl, cycloheteroalkyl, alkanoylamino, alkylamido, arylcarbonyl-amino, nitro, cyano, thiol, alkylthio, and/or any of the alkyl substituents set out herein.

Unless otherwise indicated, the term "alkynyl" as used herein by itself or as part of another group refers to straight or branched chain radicals of 2 to 20 carbons, preferably 2 to 12 carbons, preferably 2 to 11 carbons, preferably 2 to 10 carbons, preferably 2 to 9 carbons, and more preferably 2 to 8 carbons in the chain, which include one triple bond in the normal chain, such as 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, 3-nonynyl, 4-decynyl,3-undecynyl, 4-dodecynyl, and the like, and which may be optionally substituted with 1 to 4 substituents, namely, halogen, haloalkyl, alkyl, alkoxy, alkenyl, alkynyl, aryl, arylalkyl, cycloalkyl, amino, heteroaryl, cycloheteroalkyl, hydroxy, alkanoylamino, alkylamido, arylcarbonylamino, nitro, cyano, thiol, and/or alkylthio, and/or any of the alkyl substituents set out herein.

Unless otherwise indicated, the term "cycloalkyl" as employed herein alone or as part of another group includes saturated or partially unsaturated (containing 1 or 2 double bonds) cyclic hydrocarbon groups containing 1 to 3 rings, including monocyclic alkyl, bicyclic alkyl (or bicycloalkyl) and tricyclic alkyl, containing a total of 3 to 20 carbons forming the ring, preferably 3 to 10 carbons, forming the ring and which may be fused to 1 or 2 aromatic rings as described for aryl. The "cycloalkyl" containing one ring preferably contains 3 to 8 ring carbon atoms, preferably 3 to 7 ring carbon atoms, and more preferably 3 to 6 ring carbon atoms. The "cycloalkyl" includes cyclopropyl, cyclobutyl, cyclopentyl, cyclohexyl, cycloheptyl, cyclooctyl, cyclodecyl and cyclododecyl, cyclohexenyl, 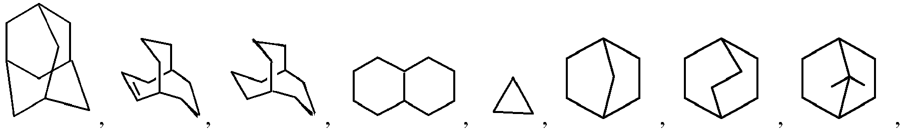 any of which groups may be optionally substituted with 1 to 4 substituents such as halogen, alkyl, alkoxy, hydroxy, aryl, aryloxy, arylalkyl, cycloalkyl, alkylamido, alkanoylamino, oxo, acyl, arylcarbonylamino, amino, nitro, cyano, thiol, and/or alkylthio, and/or any of the substituents for alkyl.

Where alkyl groups as defined above have single bonds for attachment to other groups at two different carbon atoms, they are termed "alkylene" groups and may optionally be substituted as defined above for "alkyl".

Where alkenyl groups as defined above and alkynyl groups as defined above, respectively, have single bonds for attachment at two different carbon atoms, they are termed "alkenylene groups" and "alkynylene groups", respectively, and may optionally be substituted as defined above for "alkenyl" and "alkynyl".

"Halo" or "halogen" as used herein refers to fluoro, chloro, bromo, and iodo; and "haloalkyl" is intended to include both branched and straight-chain saturated aliphatic hydrocarbon groups, for example CF₃, having the specified number of carbon atoms, substituted with 1 or more halogen (for example -CᵥF_{w} where v = 1 to 3 and w = 1 to (2v+1)).

Unless otherwise indicated, the term "aryl" as employed herein alone or as part of another group refers to monocyclic and bicyclic aromatic groups containing 6 to 10 carbons in the ring portion (such as phenyl or naphthyl, including 1-naphthyl and 2-naphthyl) and may optionally include 1 to 3 additional rings fused to a carbocyclic ring or a heterocyclic ring (such as aryl, cycloalkyl, heteroaryl, or cycloheteroalkyl rings for example 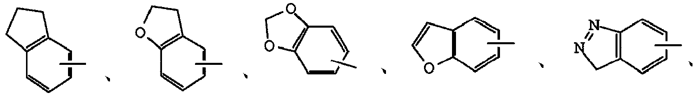 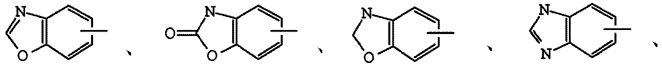 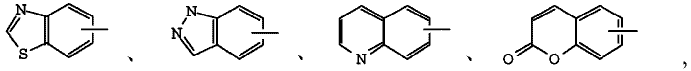 and may be optionally substituted through available carbon atoms with 1, 2, or 3 substituents, for example, hydrogen, halo, haloalkyl, alkyl, haloalkyl, alkoxy, haloalkoxy, alkenyl, trifluoromethyl, trifluoromethoxy, alkynyl, cycloalkyl-alkyl, cycloheteroalkyl, cycloheteroalkylalkyl, aryl, heteroaryl, arylalkyl, aryloxy, aryloxyalkyl, arylalkoxy, arylthio, arylazo, heteroarylalkyl, heteroarylalkenyl, heteroarylheteroaryl, heteroaryloxy, hydroxy, nitro, cyano, amino, substituted amino wherein the amino includes 1 or 2 substituents (which are alkyl, aryl, or any of the other aryl compounds mentioned in the definitions), thiol, alkylthio, arylthio, heteroarylthio, arylthioalkyl, alkoxyarylthio, alkylcarbonyl, arylcarbonyl, alkyl-aminocarbonyl, arylaminocarbonyl, alkoxycarbonyl, aminocarbonyl, alkylcarbonyloxy, arylcarbonyloxy, alkylcarbonylamino, arylcarbonylamino, arylsulfinyl, arylsulfinylalkyl, arylsulfonylamino, or arylsulfonaminocarbonyl, and/or any of the alkyl substituents set out herein.

Unless otherwise indicated, the term "alkoxy", "aryloxy" or "aralkoxy" as employed herein alone or as part of another group includes any of the above alkyl, aralkyl, or aryl groups linked to an oxygen atom.

Unless otherwise indicated, the term "amino" as employed herein alone or as part of another group refers to amino that is unsubstituted or may be substituted with one or two substituents, which may be the same or different, such as alkyl, aryl, arylalkyl, heteroaryl, heteroarylalkyl, cycloheteroalkyl, cycloheteroalkylalkyl, cycloalkyl, cycloalkylalkyl, haloalkyl, hydroxyalkyl, alkoxyalkyl, or thioalkyl. These substituents may be further substituted with a carboxylic acid and/or any of the R¹ groups or substituents for R¹ as set out above. In addition, the amino substituents may be taken together with the nitrogen atom to which they are attached to form 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, or 4-diarylalkyl-1-piperazinyl, all of which may be optionally substituted with alkyl, alkoxy, alkylthio, halo, trifluoromethyl, or hydroxy.

Unless otherwise indicated, the term "alkylthio," "arylthio," or "aralkylthio" as employed herein alone or as part of another group includes any of the above alkyl, aralkyl, or aryl groups linked to a sulfur atom.

Unless otherwise indicated, the term "alkylamino," "arylamino," or "arylalkylamino" as employed herein alone or as part of another group includes any of the above alkyl, aryl, or arylalkyl groups linked to a nitrogen atom.

As used herein, the term "heterocyclyl" or "heterocyclic system" is intended to mean a stable 5, 6, or 7-membered monocyclic or bicyclic or 7, 8, 9, or 10-membered bicyclic heterocyclic ring which is saturated, partially unsaturated or unsaturated (aromatic), and which consists of carbon atoms and 1, 2, 3, or 4 heteroatoms independently selected from the group consisting of N, NH, O and S and including any bicyclic group in which any of the above-defined heterocyclic rings is fused to a benzene ring. The nitrogen and sulfur heteroatoms may optionally be oxidized. The heterocyclic ring may be attached to its pendant group at any heteroatom or carbon atom, which results in a stable structure. The heterocyclic rings described herein may be substituted on carbon or on a nitrogen atom if the resulting compound is stable. If specifically noted, a nitrogen in the heterocycle may optionally be quaternized. It is preferred that when the total number of S and O atoms in the heterocycle exceeds 1, then these heteroatoms are not adjacent to one another. As used herein, the term "aromatic heterocyclic system" or "heteroaryl" is intended to mean a stable 5- to 7- membered monocyclic or bicyclic or 7- to 10-membered bicyclic heterocyclic aromatic ring which consists of carbon atoms and from 1 to 4 heteroatoms independently selected from the group consisting of N, O and S and is aromatic in nature.

Examples of heterocycles include, but are not limited to, 1H-indazole, 2-pyrrolidonyl, 2H,6H-1,5,2-dithiazinyl, 2H-pyrrolyl, 1H-indolyl, 4-piperidonyl, 4aH-carbazole, 4H-quinolizinyl, 6H-1,2,5-thiadiazinyl, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazalonyl, carbazolyl, 4a*H*-carbazolyl, -carbolinyl, chromanyl, chromenyl, cinnolinyl, decahydroquinolinyl, 2*H*,6*H*- 1,5,2-dithiazinyl, dihydrofuro[2,3-*b*]tetrahydrofuran, furanyl, furazanyl, imidazolidinyl, imidazolinyl, imidazolyl, indazolyl, indolenyl, indolinyl, indolizinyl, indolyl, isobenzofuranyl, isochromanyl, isoindazolyl, isoindolinyl, isoindolyl, isoquinolinyl, benzimidazolyl, isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, piperidonyl, 4-piperidonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, pyrrolyl, quinazolinyl, quinolinyl, 4*H*-quinolizinyl, quinoxalinyl, quinuclidinyl, carbolinyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, tetrazolyl, and xanthenyl. In another aspect of the present disclosure, the heterocycles include, but are not limited to, pyridinyl, thiophenyl, furanyl, indazolyl, benzothiazolyl, benzimidazolyl, benzothiaphenyl, benzofuranyl, benzoxazolyl, benzisoxazolyl, quinolinyl, isoquinolinyl, imidazolyl, indolyl, isoidolyl, piperidinyl, piperidonyl, 4-piperidonyl, piperonyl, pyrrazolyl, 1,2,4-triazolyl, 1,2,3-triazolyl, tetrazolyl, thiazolyl, oxazolyl, pyrazinyl, and pyrimidinyl. Also included are fused ring and spiro compounds containing, for example, the above heterocycles.

Examples of heteroaryls are 1H-indazole, 2H,6H-1,5,2-dithiazinyl, indolyl, 4aH-carbazole, 4H-quinolizinyl, 6H-1,2,5-thiadiazinyl, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazalonyl, carbazolyl, 4a*H*-carbazolyl, β-carbolinyl, chromanyl, chromenyl, cinnolinyl, decahydroquinolinyl, 2*H*,6*H*-1,5,2-dithiazinyl, dihydrofuro[2,3-*b*]tetrahydrofuran, furanyl, furazanyl, imidazolidinyl, imidazolinyl, imidazolyl, indazolyl, indolenyl, indolinyl, indolizinyl, indolyl, isobenzofuranyl, isochromanyl, isoindazolyl, isoindolinyl, isoindolyl, isoquinolinyl (benzimidazolyl), isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, piperidonyl, 4-piperidonyl, pteridinyl, purinyl, pyranyl, pyrazinyl, pyrazolidinyl, pyrazolinyl, pyrazolyl, pyrazolotriazinyl, pyridazinyl, pyridooxazole, pyridoimidazole, pyridothiazole, pyridinyl, pyridyl, pyrimidinyl, pyrrolidinyl, pyrrolinyl, pyrrolyl, quinazolinyl, quinolinyl, 4*H*-quinolizinyl, quinoxalinyl, quinuclidinyl, carbolinyl, tetrahydrofuranyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, tetrazolyl, and xanthenyl. In another aspect of the present disclosure, examples of heteroaryls are indolyl, benzimidazolyl, benzofuranyl, benzothiofuranyl, benzoxazolyl, benzthiazolyl, benztriazolyl, benztetrazolyl, benzisoxazolyl, benzisothiazolyl, benzimidazalonyl, cinnolinyl, furanyl, imidazolyl, indazolyl, indolyl, isoquinolinyl isothiazolyl, isoxazolyl, oxazolyl, pyrazinyl, pyrazolyl, pyrazolotriazinyl, pyridazinyl, pyridyl, pyridinyl, pyrimidinyl, pyrrolyl, quinazolinyl, quinolinyl, thiazolyl, thienyl, and tetrazolyl.

The term "cyano" as used herein refers to a -CN group.

The term "nitro" as used herein refers to a -NO₂ group.

The term "hydroxy" as used herein refers to an -OH group.

The term "mercapto" as used herein refers to a -SH group.

The term "carbamoyl" as used herein refers to -C(=O)-amino, wherein the amino group is an optionally substituted amino group.

"Alkanoyl" means a RC(=O)- group, wherein R is an alkyl group as defined herein.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "pharmaceutically acceptable salt" refers to a salt that retain the biological effectiveness and properties of the compounds of this invention and, which are not biologically or otherwise undesirable. In many cases, the compounds of the present disclosure are capable of forming acid and/or base salts by virtue of the presence of amino and/or carboxyl groups or groups similar thereto (e.g., phenol or hydroxyamic acid). Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, *p*- toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. The pharmaceutically acceptable salts of the present disclosure can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are preferred, where practicable. Lists of additional suitable salts can be found, e.g., in Remington's Pharmaceutical Sciences, 20th ed., Mack Publishing Company, Easton, Pa., (1985), which is herein incorporated by reference.

Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, maleates (formed with maleic acid), 2-hydroxyethanesulfonates, lactates, methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

Exemplary basic salts include ammonium salts, alkali metal salts such as sodium, lithium, and potassium salts; alkaline earth metal salts such as calcium and magnesium salts; barium, zinc, and aluminum salts; salts with organic bases (for example, organic amines) such as trialkylamines such as triethylamine, procaine, dibenzylamine, N-benzyl-β-phenethylamine, 1-ephenamine, N,N'-dibenzylethylene-diamine, dehydroabietylamine, N-ethylpiperidine, benzylamine, dicyclohexylamine or similar pharmaceutically acceptable amines and salts with amino acids such as arginine, lysine and the like. Basic nitrogen-containing groups may be quaternized with agents such as lower alkyl halides (*e.g.,* methyl, ethyl, propyl, and butyl chlorides, bromides and iodides), dialkyl sulfates (e.g., dimethyl, diethyl, dibutyl, and diamyl sulfates), long chain halides (*e.g.,* decyl, lauryl, myristyl and stearyl chlorides, bromides and iodides), aralkyl halides (*e.g.,* benzyl and phenethyl bromides), and others. Preferred salts include monohydrochloride, hydrogensulfate, methanesulfonate, phosphate or nitrate salts.

Prodrugs and solvates of the inventive compounds are also contemplated. The term "prodrug" denotes a compound which, upon administration to a subject, undergoes chemical conversion by metabolic or chemical processes to yield a compound as described herein, and/or a salt and/or solvate thereof. Any compound that will be converted *in vivo* to provide the bioactive agent is a prodrug within the scope and spirit of the invention. For example, compounds containing a carboxy group can form physiologically hydrolyzable esters which serve as prodrugs by being hydrolyzed in the body to yield compounds *per se.* Prodrugs include acid derivatives well know to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this disclosure are particular prodrugs. In some cases it is desirable to prepare double ester type prodrugs such as (acyloxy)alkyl esters or ((alkoxycarbonyl)oxy)alkylesters. Particularly the C₁ to Cs alkyl, C₂-C₈ alkenyl, C₂-C₈ alkynyl, aryl, C₇-C₁₂ substituted aryl, and C₇-C₁₂ arylalkyl esters of the compounds of the present disclosure.

Such prodrugs are preferably administered orally since hydrolysis in many instances occurs principally under the influence of the digestive enzymes. Parenteral administration may be used where the ester per se is active, or in those instances where hydrolysis occurs in the blood.

Various forms of prodrugs are well known in the art. For examples of such prodrug derivatives, see:
*a)* Design of Prodrugs, H. Bundgaard, ed., Elsevier(1985*), and* Methods in Enzymology, 112:309-396, K. Widder et al., eds., Academic Press(1985*);*
*b)* Bundgaard, H., Chapter 5, "Design and Application of Prodrugs," A Textbook of Drug Design and Development, pp. 113-191, P. Krosgaard-Larsen et al., eds., Harwood Academic Publishers(1991*); and*
*c)* Bundgaard, H., Adv. Drug Deliv. Rev., 8:1-38(1992*),*
each of which is incorporated herein by reference.

Esters are typically compounds derived from acids (organic acids or inorganic acids) in which at least one -OH (hydroxy) group is replaced by an -O-alkyl (alkoxy) group. Esters are usually derived from carboxylic acids and alcohols.

The esters of the compounds of the present disclosure are preferably in vivo hydrolysable esters.

As used herein, the term *"in vivo* hydrolysable ester" is understood as meaning an *in vivo* hydrolysable ester of a compound of the present disclosure containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, e.g. methoxymethyl, C₁-C₆ alkanoyloxymethyl esters, *e.g.* pivaloyloxymethyl, phthalidyl esters, C₃-C₈ cycloalkoxy-carbonyloxy-C₁-C₆ alkyl esters, *e.g.* 1-cyclohexylcarbonyloxyethyl; 1,3-dioxolen-2-onylmethyl esters, *e.g.* 5-methyl-1,3-dioxolen-2-onylmethyl; and C₁-C₆-alkoxycarbonyloxyethyl esters, e.g. 1-methoxycarbonyloxyethyl, and may be formed at any carboxy group in the compounds of this disclosure.

An *in vivo* hydrolysable ester of a compound of the present disclosure containing a hydroxy group includes inorganic esters such as phosphate esters and [α]-acyloxyalkyl ethers and related compounds which as a result of the *in vivo* hydrolysis of the ester breakdown to give the parent hydroxy group. Examples of [α]-acyloxyalkyl ethers include acetoxymethoxy and 2,2-dimethylpropionyloxymethoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The present disclosure covers all such esters.

"Solvate" refers to forms of the compound that are associated with a solvent or water (also referred to as "hydrate"), usually by a solvolysis reaction. This physical association includes hydrogen bonding. Conventional solvents include water, ethanol, acetic acid and the like. The compounds of the present disclosure may be prepared e.g. in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. "Solvate" encompasses both solution-phase and isolable solvates. Representative solvates include hydrates, ethanolates and methanolates.

A "subject" to which administration is contemplated includes, but is not limited to, humans (i.e., a male or female of any age group, e.g., a pediatric subject (e.g, infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents such as mice and rats, cats, and/or dogs. In certain embodiments, the subject is a human. In certain embodiments, the subject is a non-human animal. The terms "human," "patient," and "subject" are used interchangeably herein.

An "effective amount" means the amount of a compound that, when administered to a subject for treating or preventing a disease, is sufficient to effect such treatment or prevention. The "effective amount" can vary depending on the compound, the disease and its severity, and the age, weight, etc., of the subject to be treated. A "therapeutically effective amount" refers to the effective amount for therapeutic treatment. A "prophylatically effective amount" refers to the effective amount for prophylactic treatment.

"Preventing" or "prevention" or "prophylactic treatment" refers to a reduction in risk of acquiring or developing a disease or disorder (i.e., causing at least one of the clinical symptoms of the disease not to develop in a subject not yet exposed to a disease-causing agent, or predisposed to the disease in advance of disease onset.

The term "treating" or "treatment" refers to: (i) preventing a disease, disorder or condition from occurring in a patient that may be predisposed to the disease, disorder, and/or condition but has not yet been diagnosed as having it; (ii) inhibiting the disease, disorder, or condition, *i.e.,* arresting its development; or (iii) relieving the disease, disorder, or condition, *i.e.,* causing regression of the disease, disorder, and/or condition.

As used herein, the term "inhibition" or "inhibiting" refers to the reduction or suppression of a given condition, symptom, or disease, or a significant decrease in the baseline activity of a biological activity or process.

As used herein, the term "inhibitor" refers to a molecule which is capable of inhibiting (including partially inhibiting or allosteric inhibition) one or more of the biological activities of a target molecule, e.g., a farnesyl diphosphate synthase (FPPS). Inhibitors, for example, act by reducing or suppressing the activity of a target molecule and/or reducing or suppressing signal transduction.

The compounds described herein may exist in stereoisomeric forms (e.g., it contains one or more asymmetric carbon atoms). The individual stereoisomers (enantiomers and diastereomers) and mixtures of these are included within the scope of the present disclosure. Likewise, it is understood that the compounds described herein or salts thereof may exist in tautomeric forms other than that shown in the Formula and these are also included within the scope of the present disclosure. It is to be understood that the present disclosure includes all combinations and subsets of the particular groups defined hereinabove. The scope of the present disclosure includes mixtures of stereoisomers as well as purified enantiomers or enantiomerically/diastereomerically enriched mixtures. It is to be understood that the present disclosure includes all combinations and subsets of the particular groups defined hereinabove.

One enantiomer of the compounds described herein may exhibit superior activity as compared to another enantiomer. Therefore, all stereochemistry is considered as part of the present disclosure. The separation of racemic materials when desired may be achieved by using a chiral column for HPLC or by using a resolving agent such as camphanyl chloride for resolution (for example, as described by Young, S.D. et al., Antimicrobial Agents and Chemotherapy 1995, 2602-2605).

The subject disclosure also includes isotopically-labelled compounds, which are identical to those recited in Formula (I) and following, but for the fact that one or more atoms are replaced by an atom having an atomic mass or mass number different from the atomic mass or mass number usually found in nature. Examples of isotopes that can be incorporated into compounds of the present disclosure and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N ¹⁷O ¹⁸O ³¹P ³²P ³⁵S, ¹⁸F ³⁶Cl ¹²³I, and ¹²⁵I.

Compounds of the present disclosure and pharmaceutically acceptable salts of said compounds that contain the aforementioned isotopes and/or other isotopes of other atoms are within the scope of the present disclosure. Isotopically-labelled compounds of the present disclosure, for example those into which radioactive isotopes such as ³H or ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of Formula (I) and following of this disclosure can generally be prepared by carrying out the procedures disclosed in the Schemes and/or in the Examples below, by substituting a readily available isotopically labelled reagent for a non-isotopically labelled reagent.

### Indication

In aspects of the present disclosure, the cancer may be a malignant or non-malignant cancer. Cancers or tumors include, but are not limited to, biliary tract cancer; bladder cancer; brain cancer; breast cancer; cervical cancer; choriocarcinoma; colon cancer; colorectal cancer; endometrial cancer; esophageal cancer; gastric cancer; glioblastoma; intraepithelial neoplasms; lymphomas (for example, follicular lymphoma); liver cancer; lung cancer (for example, small cell and non-small cell); leukemia (for example, hairy cell leukemia, chronic myelogenous leukemia, cutaneous T-cell leukemia); melanoma (for example, malignant melanoma); multiple myeloma; neuroblastomas; oral cancer; ovarian cancer; pancreatic cancer; prostate cancer; rectal cancer; renal cancer; sarcomas; skin cancer; testicular cancer; thyroid cancer; and kidney cancer, as well as other malignancies and sarcomas (for example, squamous cell tumor, renal cell tumor, prostate tumor, bladder tumor, or colon tumor).

### Administration mode and dose

For use in therapy, an effective amount of one or more immunogenic compositions may be administered to a subject. Administering the pharmaceutical composition of the present disclosure may be accomplished by any means known to the skilled artisan. Preferred routes of administration include but are not limited to parenteral (for example, intramuscular, subcutaneous, intradermal, intravenous injection), topical to the skin (for example, transdermal) or mucosal (for example, oral, intranasal, intravaginal, intrarectal, trans-buccal, intraocular or sublingual). In the case of treatment of cancers, this may include intra-tumor administrations.

In some aspects of the present disclosure, an "effective amount" of an immunogenic composition refers to an amount that is necessary or sufficient to achieve the desired biological effect. For example, an effective amount of an immunogenic composition for treating a condition may be an amount necessary to eliminate a microbial infection or a tumor. An effective amount for use as a vaccine adjuvant may be an amount that can be used to enhance the immune response of a subject to a vaccine. The effective amount may vary depending on the following parameters: the disease or condition to be treated, the particular immunogenic composition administered, the age of the subject or the severity of the disease or condition. One of ordinary skill in the art can empirically determine the effective amount of a particular immunogenic composition without undue experimentation.

The immunogenic composition may be administered in a single dosage regimen, or preferably in a plurality of dosage regimens. That is, the main process of inoculating the immunogenic composition is 1-10 individual doses and subsequent administration of other doses at subsequent intervals according to the need to maintain and/or enhance the immune response, for example, a second dose after 1-4 months, and, if desired, a subsequent dose after a few months or years. The medication regimen is also determined at least in part by the needs of the individual and depends on the judgment of the medical staff. Examples of suitable immunization regimens include: a first dose, followed by a second dose between day 7 and month 6, and optionally a third dose between month 1 and year 2 after the first inoculation; or other schemes that are sufficient to elicit a virus neutralizing antibody titer which is desirable for imparting protective immunity, for example, correspond to an established inoculation scheme for a pediatric immunogenic composition. Satisfactory protective immunity can be maintained by supplementing the enhanced dose given at specific intervals (e.g., every two years).

The immunogenic compositions of the present disclosure can be prepared in various forms such as injections, tablets, powders, granules, capsules, oral solutions, unguentums, creams and the like. The above-mentioned various dosage forms of the medicament may be prepared according to conventional methods in the field of pharmacy. One or more pharmaceutically acceptable carriers may also be added to the formulations described above. The carrier includes conventional diluents, excipients, fillers, binders, wetting agents, disintegrating agents, absorption enhancers, surfactants, adsorption carriers, lubricants and the like in the pharmaceutical field.

When it is desired to systemically deliver one or more immunogenic compositions, they may be formulated for parenteral administration by injection (e.g., bolus or instillation). For example, the subject's sole, subcutaneous, muscle, abdominal and nasal mucosa can be injected to immunize. The formulation for injection may be presented in unit dosage form, for example, in an ampoule or in a multi-dose container with the added preservative. For example, the compositions may take the form of suspensions, solutions or emulsions in oily or aqueous vehicles and may contain formulation agents such as suspending agents, stabilizers and/or dispersing agents.

### EXAMPLES

The following examples are provided to provide those skilled in the art with a complete disclosure and description of how to make, prepare, and evaluate the methods and compounds claimed herein, and are intended to merely exemplify the invention without limiting the scope of the invention.

### Biological examples

All data in the biological examples were statistically analyzed using Graphpad Prism5 software. The measured data was presented in the form of mean ± standard error (x ± SEM). The comparison of the means between two groups was performed by t test. The comparison of the means between the control group and experimental groups (inter-group comparison) was performed by one-sided ANOVA analysis. *: P <0.05, **: P <0.01, ***: P <0.001, ns: no significant difference (not significant).

### Example 1

### Mevalonate pathway inhibitors cause immunogenic cell death (ICD) of tumor cells and promote the body's immune response

Apoptosis is a way of cell death. Apoptosis can be divided into physiological death and immunogenic cell death (ICD) according to whether the surface of apoptotic cells can express the protein molecules that cause the body's immune attack. The tumor cells when treated by high temperature, radiation, chemotherapeutic drugs and other means to cause the apoptosis, express protein molecules on the cell surface that cause the body's immune attack, causing an anti-tumor response, called ICD. Calreticulin (CRT), heat shock protein (HSPs), endoplasmic reticulum proteins (ERp), high-mobility group box 1 (HMGB-1), and other characteristic protein molecules, which are highly expressed on the surface of tumor cells which underwent immunogenic cell death, can stimulate the body's immune cells to recognize and attack tumor cells, and simultaneously release ATP chemotactic macrophages and DC cells aroud the tumor.

Mevalonate pathway inhibitors (such as statins and bisphosphonates) can cause apoptosis, but it has not been reported whether they can cause immunogenic cell death of tumor cells. We designed experiments, such as the expression experiment of CRT and ERp57 on the surface of tumor cells, experiment of phagocytosis by bone marrow-derived DC cells (BMDCs), experiment of testing whether tumor cells as tumor vaccination can protect the body against re-challenge of tumor cells, and experiment of detecting T lymphocyte infiltration in the tumor microenvironment, to detect the situation after the treatment with statins and bisphosphonates. It was thus verified that mevalonate pathway inhibitors can cause ICD.

### 1.1 Statins and bisphosphonates induce apoptosis and endoplasmic reticulum stress

Materials and Sources: Trypsin was purchased from Life technologies, DMEM and RPMI medium 1640 was purchased from Shanghai Life Biotechnology Co., Ltd., and penicillin-streptomycin solution (PS) was purchased from Beyotime Institute of Biotechnology, Fetal bovine serum (FBS) was purchased from Gemini, apoptosis kit was purchased from ebioscience, B16F10 cells were donated by Professor Liao Xuebin of Tsinghua University, and qPCR SYBR Green Master Mix products were purchased from Beijing ComWin Biotech Co., Ltd.

Method: 1×10⁵ B16F10 cells were seeded in a 6-well plate, which were treated by drugs after adhesion overnight: blank control group; Sim (simvastatin), 2 µM for 24 h; Zol (zoledronic acid), 10 µM for 48 h. After the drug action, the cells were digested with trypsin into a single-cell suspension, washed with PBS, and stained with Annexin V and PI according to the steps of the apoptosis kit. After staining, data was collected by a flow cytometer, and the data was analyzed by FlowJo7.6. Total RNA was extracted from cells according to the Trizol method, and the RNA was used as a template, and the RNA was reverse transcripted using a reverse transcription kit to cDNA. The obtained cDNA was used for real-time quantitative PCR. 10 µl reaction system was configured according to qPCR SYBR Green Master Mix product instructions, three replicates were set up for each sample. The experimental data was collected using a fluorogenic quantitative PCR instrument LightCycler480 of Roche, and the data was calculated according to the 2^{-ΔΔc(t)} method. The primer sequences used in this experiment are listed in Table 2.

**Table 2 Q-PCR primer sequences**

| Primer Name | Primer sequence (5'-3') |
|---|---|
| GRP78 F-mus | CTGGGTACATTTGATCTGACTGG |
| GRP78 R-mus | GCATCCTGGTGGCTTTCCAGCCATTC |
| ATF6 F-mus | CCCAAGACTCAAACCAAT |
| ATF6 R-mus | CTCTGACACCACCTCGTC |
| IRE1 F-mus | GAAGACGTCATTGCACGTGAATT |
| IRE1 R-mus | AGGTCCTGAATTTACGCAGGT |
| PERK F-mus | AGCGCGCAGATCACAGTCAGGTT |
| PERK R-mus | CTCACGGAGTCGTATTTACTTTCAG |

Both simvastatin and zoledronic acid can cause apoptosis of B16F10 cells. Compared with the control group, the proportion of cells in the late stage of apoptosis (Annexin + PI +) increased to about 50% at 60 hours after the drug treatment (Figure 1A, 1B). At the same time, simvastatin and zoledronic acid can also cause the increase of wrongly or incorrectly folded cellular proteins, leading to a significant increase in the expression of Grp78 and ATF6, IRE1, and PERK involved in the signal pathway of endoplasmic reticulum stress in cells. (Endoplasmic reticulum stress: its role in disease and novel prospects for therapy. Scientifica (Cairo) (2012) 2012:857516) (Figure 1C).

### 1.2 Statins and bisphosphonates cause the expression of CRT and ERp57 on the surface of tumor cell membrane

Materials: Antibodies CRT (ab2907) and ERp57 (ab10287) were purchased from Abcam. Goat-anti-rabbit IgG-AF-488 was purchased from Beijing Zhongshan Golden Bridge Biotechnology Co., Ltd.

### Methods:

In vitro experiments: 1 × 10⁵ B16F10 cells were seeded in a 6-well plate, treated with Sim 10 µM for 24 h, and Zol 20 µM for 60 h. After the drug treatment, the cells were digested with trypsin into a single-cell suspension, washed with PBS and stained. Staining buffer formula: PBS containing 0.5% bovine serum albumin (BSA), 2 mM EDTA. About 1 × 10⁵ cells were taken for staining analysis. First, the cells were stained with primary antibody, 100 µl of anti-CRT (1: 200 dilution) and anti-ERp57 (diluted at 1: 200), for 1 h on ice; washed with 1 ml of PBS after staining; stained with secondary antibody, Goat-anti-rabbit IgG-AF488 (1: 1000 dilution), for 1 h; wash with 1 ml PBS; resuspend in 100 µl buffer after washing, detected by a flow cytometer within 1 h. 100 µl fluorescent antibody storage solution (100 ml PBS + 1 ml formaldehyde + 2 g glucose) may also be used for resuspension and detected by a flow cytometer within 72 hours. Data were analyzed with FlowJo7.6.

In vivo experiments: 1 × 10⁶ 4T1 cells were transplanted subcutaneously into the left hip of BALB/c mice and administered starting on the 8th day after tumor transplantation: Sim, dissolved in 75% DMSO, 2 mg/kg, intratumoral injection (i.t.), once every two days for a total of 3 times; Zol, dissolved in sterile PBS, 1 mg/kg, i.t. for once. Tumors were harvested on the 14th day after tumor transplantation. Tumor dissociation kit (mouse) was used to dissociate the tumor into single cells for staining. The staining procedure was performed according to the in vitro experiments as mentioned above.

Next, we investigated whether simvastatin and zoledronic acid can induce tumor cell death and induce the eversion of endoplasmic reticulum chaperone molecules such as CRT and ERp57 to the cell membrane surface at the same time. We used B16F10 mouse melanoma cells with very weak immunogenicity as tool cells. After treatment with Zol and Sim in vitro, the CRT and ERp57 on the cell membrane surface was detected by an indirect staining method, finding out that Zol and Sim treatment significantly promoted the expression of CRT (Figure 2A) and ERp57 (Figure 2B) on the membrane surface, which was about twice that of the control group. Subsequently, we used 4T1 mouse breast cancer cells to verify in vivo. The experimental dosing regimen is shown in Figure 2C. Consistent with our in vitro data, intratumoral injection of Zol and Sim can also promote the exposure of tumor cell epitopes. The CRT and ERp57 expressed on the membrane surface were about 1.7 times that of the control group (Figures 2D, 2E).

### 1.3 Statins and bisphosphonates promote tumor cells to express type I interferon (IFN-I) and type I interferon stimulated genes (IFN-I-stimulated genes, ISGs)

Type I interferons, including IFN-α and IFN-β, are the major effector molecules involved in antiviral process. When the surface or internal receptors (Toll-like receptors, NOD-like receptors, RIG-I receptors, cGAS, etc.) of the innate immune cell contact with virus-specific antigen substances (DNA, RNA), the intracellular signal molecules (MAVS, STING, TBK, IKK, etc.) are passed through, thereby activating the downstream pathway to release IFN-I. In recent years, some researchers have found that after being treated with anthracycline, the dying tumor cells release RNA, and this RNA is recognized by the Toll-like receptor 3 (TLR3) of the surrounding undead tumor cells to release IFN-I and ISGs, recruiting immune cells to the tumor microenvironment in a TLR3-IFNs-I-IFNAR-CXCL10 signaling mode to kill tumor cells (Inhibiting DNA Methylation Causes an Interferon Response in Cancer via dsRNA Including Endogenous Retroviruses, Cell. 2017 Apr 6;169(2):361). In addition, some researchers have called the way that drugs promote tumor cells to release IFN-I and ISGs as a type of ICD response (Autocrine signaling of type 1 interferons insuccessful anticancer chemotherapy, Oncoimmunology. 2015 Jul 25;4(8):e988042).

Materials and Sources: Trizol was purchased from Beijing ComWin Biotech Co., Ltd.; LightCycler® 480 II instrument was purchased from Roche. Anti-PDL-1-FITC antibody was purchased from Biolegend.

Method: B16F10 cells were cultured with RPMI medium 1640 + 10% FBS + 1% PS, and the remaining cells were cultured with DMEM + 10% FBS + 1% PS. 5x10⁵ B16F10 and 4T1 cells were plated in 6-well plates and adhered for 12 hours, and then treated with simvastatin and zoledronic acid in the figure, and the control group was treated with normal culture medium. A part of the cells treated with the drugs were lysed with 1 ml of Trizol, and RNA was extracted. The expression of IFN-β1, IRF7, IFI44L, IL-15, MAGEB-2 and other genes was detected by LightCycler® 480 II using a Q-PCR method (see table 3 for the primer sequence). In addition, the rescue effect of GGOH on the role of simvastatin and zoledronic acid to promote the release of type I interferon (IFN-β1) and the expression of type I interferon-related stimulated genes ISGs was also tested.

**Table 3 Q-PCR primer sequences**

| Primer Name | Primer sequence (5'-3') |
|---|---|
| 1fN-β1-F-mus | AGCTCCAAGAAAGGACGAACA |
| IfN-β1-R-mus | GCCCTGTAGGTGAGGTTGAT |
| IRF7-F-mus | GCGTACCCTGGAAGCATTTC |
| IRF7-R-mus | GCACAGCGGAAGTTGGTCT |
| IFI27-F-mus | TCAGATTTTCCTTCCCCGCA |
| IFI27-R-mus | TACTGTCATGGCTCCCCCAA |
| IFI44L-F-mus | GCTGCGGATGATGCCTTAGA |
| IFI44L-R-mus | TGAGGGTTTCCCAAAAGGCA |
| MAGEB2-F-mus | TCCCTAGTGCCTTCCCTAATCT |
| MAGEB2-R-mus | GTTAGAGGACTTTTGGGATGGGG |
| GAPDH F-mus | TGACCACAGTCCATGCCATC |
| GAPDH R-mus | GACGGACACATTGGGGGTAG |
| IL15-F-mus | CATCCATCTCGTGCTACTTGTG |
| IL15-R-mus | GCCTCTGTTTTAGGGAGACCT |

Mevalonate pathway inhibitors statins simvastatin and bisphosphonates zoledronic acid can cause different degrees of the expression of type I interferon (IFN-β1), type I interferon-related stimulated genes ISGs (IFI27, IFI44), and the upstream important gene of type I interferon pathway IRF7 in mouse colon cancer cell MC38 and mouse melanoma cell B16F10. At the same time, they can promote IL-15 (promote host NK cell activation) and MAGEB2 (antigen presentation) expression. And the expression of the above genes increased significantly at the later stage of drug action (72h) (Figures 3A, 3B). Because GGOH is the raw material for the synthesis of GGPP, and simvastatin and zoledronic acid inhibit the mevalonate pathway to inhibit GGPP synthesis, it is speculated that GGOH can counteract the effect of simvastatin and zoledronic acid on the release of type I interferon and ISG. The results in Figures 3C and 3D show that GGOH can counteract the release of type I interferon and ISG by simvastatin and zoledronic acid. Therefore, simvastatin and zoledronic acid can inhibit the production of GGPP by inhibiting the mevalonate pathway, promoting the release of type I interferons and ISG.

### 1.4 Statins and bisphosphonates can promote tumor cells phagocytosed by bone marrow-derived dendritic cells (BMDC)

Materials and Sources: CellTracker™ Red CMTPX was purchased from Life technology, CFSE was purchased from ebiscience, C57/b6 mice were purchased from Charles River Experimental Animal Technology Co., Ltd., GM-CSF cytokine granulocyte-macrophage colony-stimulating factor, and IL-4 were purchased from eBioscience, and doxorubicin (DOX) was purchased from Energy chemical company.

Method: C57/b6 mouse bone marrow cells were cultured in DC differentiation solution, RMPI 1640 + 10 ng/ml GM-CSF + 10 ng/ml IL-4 containing 10% FBS for 7 days. It was ready for use when the percentage of DC cells was above 90%.

B16F10 cells were plated in 6-well plates at a density of 5x10⁵. After being adhered for 12 hours, they were treated with Sim and Zol in the figure, and the control group was treated with normal culture medium. The cells treated with the drug were digested with trypsin, and the cells were obtained after centrifugation.

Cell Staining: Flow Cytometry Staining: B16F10 tumor cells were stained for 30 min in 1 ug/ml CFSE at room temperature, and washed twice with PBS after staining; BMDC cells were stained for 30 min in 1 uM CellTracker™ Red, and washed twice with PBS after staining. Cells were ready for use. Immunofluorescence staining: B16F10 cells were stained with DiD, and DC cells were stained with CFSE.

After the drug treatment, the B16F10 control group Ctrl, Sim treatment group, Zol treatment group, and DOX treatment group were co-incubated with DC cells in a 1:1 ratio for 3 hours in an incubator at 37 °C, and fixed with 200ul fluorescent antibody preservation solution (1 ml formaldehyde, 2 g glucose dissolved in 100 ml PBS) after the incubation. Flow cytometer BD FACSAria SORP was used for detection. Data was analyzed with Flojo 7.6.2 software. Cell Tracker™ Red-positive DC cells were first circled out, and the number of CellTracker™ Red-positive DC cells phagocytosing CFSE-positive tumor cells was analysized. After the immunofluorescence staining, the phagocytosis of tumor cells (red) by DC cells (green) was observed with zeiss laser confocal microscope LSM710META.

After bone marrow-derived cells were cultured in DC differentiation solution for 7 days, CD11c + DC cells accounted for more than 90% of the total cells. Dox has been reported to cause ICD of tumor cells (Calreticulin exposure dictates the immunogenicity of cancer cell death, Nat Med. 2007 Jan;13(1):54-61), which was served as a positive control in this experiment. Among B16F10 cells, 10% of DC cells phagocytosed tumor cells in the control group without drug treatment; tumor cells treated with dox were more likely to be endocytosed by DC cells, approximately 38% of DC cells phagocytosed tumor cells; for Zol treatment, about 35% of DC cells phagocytosed tumor cells; while for Sim treatment, 51.6% of DC cells phagocytosed tumor cells, which is higher than that of the dox group (Figure 4A). And it can be seen that after being treated with statins and bisphosphonates, tumor cells are more easily phagocytosed by DC cells. Immunofluorescence data showed that green DC cells phagocytosed more red tumor cells in the Sim and Zol dosing group than that in the control group (Figure 4B), and it can be seen from the images that the scattered red dot distribution in green DC cells in the dosing group indicates that the DC cells have digested the tumor cells (Figure 4B).

### 1.5 Tumor cells treated with statins and bisphosphonates can be used as a tumor vaccine to protect the body against the re-challenge of tumor cells

Method: CT26 cells were treated with simvastatin or zoledronic acid. 1.5-3x10⁶ dying cells were implanted subcutaneously in the right side of BALB/c mice. The control group was injected with PBS. 1 × 10⁶ live CT26 cells were implanted subcutaneously in the left side of the mice, and tumor volume was observed and measured. Mice with tumor volumes > 100 mm³ were identified as tumor-growing mice, and the number of mice without tumor growth was recorded.

A key factor to determine the ability of drugs to induce immunogenic cell death is whether drug-treated tumor cells can be used as vaccines to protect the body against the re-challenge of the tumor cells. We treated CT26 mouse colon cancer cells with Sim and Zol for 72 h to a dying state, and BALB/c mice were immuned with these dying cells, wherein PBS was injected in the control group. After 7 days, the mice were injected with 5 × 10⁵ live CT26 cells (re-challenge), and the tumor growth was observed after 7 days. 100% of the mice in the control group (n = 10) grew tumors on day 9. After 30 days of transplantation, in mice immunized with CT26 cells treated with Sim (n = 10) and Zol (n = 10), the ratios of tumor-free mice were 80% and 90%, respectively, with statistical difference (Figure 5).

### Example 2

### Mevalonate pathway inhibitors promote DC immune response

### 2.1 Statins and bisphosphonates can promote cross-presentation of DC cells to cause T-cell responses

Materials and Sources: OT-I and OT-II transgenic mice were donated by Professor Shi Yan of Tsinghua University, BALB/c mice were purchased from Charles River Co., Ltd., a CD4 and a CD8a sorting magnetic beads were purchased from German Miltenyi company. CFSE dye, mouse IFN-γ and TNF-α were purchased from eBioscience. CD3-APC antibody, CD4-PE antibody, and CD8-PE antibody were purchased from Bioledend, USA.

Method: BALB/c mouse bone marrow cells were cultured with RMPI 1640 + 10 ng/ml GM-CSF + 10 ng/ml IL-4 for 7 days to obtain BMDC. BMDC cells were treated with the medium control, Sim 1 µM and Zol 5 µM for 24 h, and the medium was washed away after the drug action, and ready for use. The spleen cells of OT-I and OT-II transgenic mice were sorted with a CD4 and a CD8a magnetic beads, respectively. CD4 cells and CD8 cells were obtained and stained with CFSE. After that, 5 × 10⁴ CFSE-stained CD4 cells and CD8 cells were co-cultured with 5 × 10⁴ BMDC cells under the condition of OVA (100 µg/mL) for 72 h. After the culture was completed, the secretion of IFN-γ and TNF-α in the supernatant was detected, and the cells were stained with CD3 + CD4 + and CD3 + CD8 + antibodies, respectively, and the cellular CFSE was detected by a flow cytometer to determine the proliferation.

CD4 cells and CD8 cells derived from OT-I and OT-II transgenic mice can specifically recognize OVA protein and activate, proliferate, and release cytokines such as IFN-γ and TNF-α. DC cells can present OVA proteins to CD4 cells and CD8 cells. Compared with the OVA-treated group alone, DC cells treated with Sim and Zol had an enhanced ability to present OVA, the proliferation of CD4 cells and CD8 cells was significantly enhanced (Figure 6A), and the release of cytokines was also significantly increased (Figure 6B).

### 2.2 Simvastatin and zoledronic acid promote adoptive DC cells infusion to treat tumors

Materials and Sources: BALB/c mice were purchased from Charles River Co., Ltd. and TRP2 was purchased from Anaspec.

Method: 2.5 × 10⁵ B16F10 cells were implanted subcutaneously in the left side of C57BL/6 mice. On the 3rd and 7th day after tumor implantation, 1 × 10⁶ DC cells were injected into the left footpads. The mice were divided into 4 groups according to different treatment methods of DC cells: control group, DC cells were not treated; TRP2 group, DC cells were incubated with 50 µg/ml TRP2 protein for 8 h; Zol + TRP2 group, DC cells were first incubated with 5 µM Zol for 24 h, and then with 50 µg/ml TRP2 protein for 8 h; Sim + TRP2 group, DC cells were first incubated with 1 µM Sim for 24 h, and then with 50 µg/ml TRP2 protein for 8 h. Tumor volume was measured every 3-4 days after two DC cells immunizations.

B16F10 cells expressed the TRP2 antigen. Bone marrow-derived DC cells were pretreated with Zol and Sim for 24 h, and then with the antigen TRP2 for 8 h. Then, the antigen-loaded DC cells were reinfused to the B16F10 tumor-bearing mice mouse immunized in foodpads. Mice in the group of DC cells pretreated with Zol and Sim showed significantly smaller tumor size than those in the Ctrl wherein DC cells were not treated and TRP2 group (Figure 7).

### Example 3

### Antitumor activity of mevalonate pathway inhibitors at low-dose depends on the host T lymphocytes and DC cells

Mevalonate pathway inhibitors statins and bisphosphonates have been reported to have antitumor activity in syngeneic tumor-bearing mice (A combination therapy for KRAS-driven lung adenocarcinomas using lipophilic bisphosphonates and rapamycin, Sci Transl Med. 2014 Nov 19;6(263):263ra161.; Simvastatin reduces melanoma progression in a murine model, Int J Oncol. 2013 Dec;43(6): 1763-70), but whether their antitumor activity depends on the mouse's own immune system has not been reported.

The immune system of the BALB/c strain mice is intact, while the BALB/c nude mouse strain lacks T lymphocytes. If a drug exhibits antitumor activity in BALB/c mice while not activity in BALB/c nude mice, the antitumor activity of the drug is likely to be exerted by stimulating T lymphocytes. Mouse-derived tumor cells were transplanted subcutaneously into the left side of BALB/c mice and BALB/c nude mice with the same background of the tumor cells, respectively. After the tumor volume reached 100 mm³, administration was started. The effect of the drug was evaluated by tumor volume.

Materials and Sources: Simvastatin and zoledronic acid were purchased from Tianjin Heowns Biochemical Technology Co., Ltd, the experimental mice were purchased from Beijing Charles River Experimental Animal Technology Co., Ltd. and kept at SPF Animals Rooms of Experimental Animal Center of Tsinghua University; CT26 cells were purchased from the Cell Bank of the Chinese Academy of Sciences, A20 cells and 4T1 cells were donated by Professor Liu Wanli and Professor Xiao Bailong of Tsinghua University; PBS and DMSO were purchased from Beijing Solarbio Technology Co., Ltd.; anti-CD8 and anti-CD4 neutralizing antibodies, anti-CD45-Percp, anti-CD3-APC, anti-CD8-PE were purchased from Biolegend; vernier calipers were purchased from JD Mall.

### 3.1 Tumor activity of statins and bisphosphonates at low-dose is associated with T cells

Test method: 1x10⁶ CT26, 4T1 and A20 cells were inoculated subcutaneously in the left side of 6-week-old female BALB/c mice and BALB/c nude mice, respectively. After the tumor volume reached 100 mm³ (about 7 days), administration was started (the tumor volume is measured using a vernier caliper. The tumor volume was calculated using formula of length (mm) × width (mm) × width (mm)/2). The tumor volume was measured every 3 to 4 days. Zoledronic acid was dissolved in PBS, and administered once at a dose of 1 mg/kg every 6 days; simvastatin was dissolved in 75% DMSO, and administered once at a dose of 2 mg/kg every two days; the control group was treated with an equal volume of PBS or 75% DMSO. After the tumor volume reached 1600 mm³, the mice were euthanized.

As can be seen from Figures 8A, 8B, and 8C, in subcutaneous xenograft tumor mouse models of A20, 4T1, and CT26 cells, zoledronic acid and simvastatin have significant antitumor activity in immune-intact mice, while the antitumor activity disappeared in nude mice without T cells, suggesting that the antitumor activity of mevalonate pathway inhibitors zoledronic acid and simvastatin depends on T lymphocytes.

### 3.2 The antitumor activity of statins and bisphosphonates at low-dose is blocked by CD8 antibody or CD4 antibody

Test method: 1x10⁶ CT26 cells were inoculated subcutaneously in the left side of 6-week-old female BALB/c mice, and grouping and administration were started on day 7 after implantation (i.p.). Tumor volume was measured every 3 to 4 days. Dosage regimen: control group: solvent plus Isotype antibody; zoledronic acid administration group: 1 mg/kg, once every 6 days for a total of two times; simvastatin administration group: 2 mg/kg, once every two days for a total of 4 times; CD8 neutralizing antibody + zoledronic acid administration group: CD8 neutralizing antibody, 50 ug/mouse, 1 hour later Zol was injected, and this combination was administered once every six days for a total of two times; CD8 neutralizing antibody + Sim administration group: CD8 neutralizing antibody, 50 ug/mouse, 1 hour later Sim was injected, antibody was administered every 6 days for a toal of two times, Sim dissolved 75% DMSO and administered once at a dose of 2 mg/kg every two days for a total of 4 times. In addition, the CD4 antibody plus Zol administration group: CD4 neutralizing antibody, 50 ug/mouse, 1 hour later Zol was injected. This combination was administered once every six days for a total of two times.

In immune-intact mice of CT26 mouse subcutaneous xenograft model, CD8 antibody was used to block the effect of CD8 T cells in mice, and CD4 antibody was used to block CD4 T cells. The antitumor activity of zoledronic acid and simvastatin disappeared in mice injected with CD8 antibody, and the tumor volume was even slightly larger than that of the non-administration control group (Figure 9A). In addition, the antitumor activity of zoledronic acid disappeared in mice injected with CD4 antibody at the same time (Figure 9B). Based on the data of the above two animal models, it is found that the antitumor activity of zoledronic acid and simvastatin depends on CD8 T and CD4 T lymphocytes.

### 3.3 statins and bisphosphonates can promote tumor infiltration of T cells and DC cells

The experimental process is shown in Figure 10A. 1 × 10⁶ 4T1 cells were injected subcutaneously into the left hip of BALB/c mice to establish a 4T1 subcutaneous model of mouse breast cancer. Seven days later, administration was started (Figure 10A). After the administration, tumors were collected on the 21st day after tumor transplantation, and tumors were digested into single-cell suspensions with a tumor dissociation kit, and then the cells were stained accordingly. Flow cytometry analysis found that in the Zol and Sim administration groups, the number of tumor infiltrating CD86 positive DC cells (Figure 10B), CD8 positive T lymphocytes (Figure 10C), and CD4 positive T lymphocytes (Figure 10D) increased, and there was statistical significance compared with the control group.

### 3.4 Anti-tumor metastasis effect of Simvastatin and zoledronic acid depends on T lymphocytes

A mouse model of in situ 4T1 breast cancer was used as a tumor metastasis model. Luci-4T1 cells were seeded into the mammary fat pads of BALB/c mice with normal immunity and BALB/c nude mice, respectively. After about 7 days, the mice were evenly divided into groups according to the luciferase luminescence value and administered. In-vivo imaging was performed every three days on the 15th day after administration to observe the tumor metastasis. The experiment found that the growth and metastasis rate of the in situ tumor in nude mice were faster than those of mice with normal immunity, which was consistent with the fact that nude mice had immunodeficiency. On the 21st day of administration, nude mice had developed systemic tumor metastasis, and the control group (without administration), simvastatin, and zoledronic acid administration group showed no difference in systemic metastasis among groups (Figure 11A); After the imaging, the mice were euthanized, the lungs were taken out and immersed in Luciferin substrate, and the tumor growth of the lungs was continued to be photographed with a in-vivo imager. As can be seen from Figure 11B, there was no significant difference in lung metastasis between the simvastatin and zoledronic acid groups compared with the control group, and the number of lung metastatic foci was also not significantly different among groups (Figure 11C). As a result, simvastatin and zoledronic acid have no anti-tumor metastatic effect in nude mice without T lymphocytes. On the 28th day after administration, the control group of BALB/c mice showed significant systemic metastases. Sim group showed very weak metastasis signs under the same imaging conditions, while Zol group mice did not show metastasis signs (Figure 11A); Similarly, the metastasis in the lungs for the Sim and Zol groups was significantly lower than that of the control group (Figures 11B, 11C). Thus, simvastatin and zoledronic acid have antitumor effects in tumor-bearing mice with normal immunity. In summary, the anti-tumor metastatic effect of simvastatin and zoledronic acid is dependent on T lymphocytes.

In addition, we continued to validate the anti-tumor metastatic effect of simvastatin and zoledronic acid using the B16F10 cells lung metastasis model. B16F10 cells were injected into BALB/c mice via tail vein. 7 days later, administration was started. On the 25th day after administration, lung tissue was removed. Figure 12 shows that Zol and Sim can significantly inhibit the lung metastasis of B 16F10 cells compared to the control group.

### 3.5 Antitumor effect of Zoledronic acid for in situ tumor depends on host DC cells

CD11c-positive DC cells in the body would be cleared after CD11c-DTR/EGFP mice were injected with DT. We established a MC38 subcutaneous tumor model in CD11c-DTR/EGFP mice to investigate whether DT clearing DC impact the antitumor effect of Zol. Figure 13A is an experimental dosing regimen; Figure 13B shows that in a MC38 homologous subcutaneous tumor model of CD11c-DTR/EGFP mice, DT can offset the anti-tumor effect of Zol, which shows that DC cells participate in the anti-tumor effect of Zol.

### Example 4

### Statins and bisphosphonates combined with immune checkpoint inhibitor PD-1 antibodies have combined antitumor effects

In recent years, PD-1/PDL-1 pathway inhibitors have shown good therapeutic effects in clinical treatment of various tumors. It has been reported in the literature that tumor cells and tumor-associated antigen-presenting cells (APC) in the tumor microenvironment including DCs cells (dendritic cells), tumor associated macrophages (TAM), fibroblasts, T cells and others, highly expressed PDL-1 (PD-L1 (B7-H1) and PD-1 pathway blockade for cancer therapy: Mechanisms, response biomarkers, and combinations, Sci Transl Med. 2016 Mar 2;8(328):328rv4). PD-L1 can be induced by a variety of cytokines. Interferon secreted by tumor-infiltrating T cells can promote tumor cells and tumor microenvironmental APC cells to overexpress PDL-1. Conversely, tumor cells overexpress PDL-1 to promote T cell death and exert immune escape effects. It can be seen that enhancing the body's immune response, promoting tumor infiltrating of CD8 + T cells, and the combination with anti-PD-1/PDL-1 immune checkpoint inhibitors will have a synergistic antitumor effect.

Materials and Sources: PD-1 antibody was purchased from BioXcell; PLV-Luci-GFP, Ps PAX, PMD 2G plasmids were donated by Professor Du Yanan of Tsinghua University; luciferin was purchased from SYNCHEM; IVIS Spectrum, Quantum FX microCT in-vivo imaging system was purchased from PerkinElmer.

Method: A luciferase-expressing 4T1 stable transfected cell line (Luci-4T1) was constructed. Luci-4T1 cells were transplanted into the mammary fat pads of homologous mice; B16F10 and MC38 cells were transplanted subcutaneously in homologous mice, respectively; administration was started when the tumor volume reached 100 mm³, Sim, 2 mg/kg, i.p., once every two days; TH-Z 93/Zol, 1 mg/kg, i.p., once every 5-7 days; PD-1 antibody, 200 ug/mouse, i.p., once every three days.

The synergistic effect of the drug is expressed as the Coefficient of Drug Interaction (CDI), which is calculated as CDI = (AB / A × B), A and B are two single drugs, AB is the combination of single A and single B. Additive effect: CDI = 1; Synergistic effect: CDI <1; Antagonistic effect: CDI> 1. (Acetazolamide potentiates the anti-tumor potential of HDACi, MS-275, in neuroblastoma, BMC Cancer. 2017 Feb 24;17 (1)). This calculation method was used for the subsequent calculating of combination effects of drugs.

In the B16F10 mouse tumor model, the single Sim, Zol, and PD-1 all showed certain antitumor activity. In addition, Sim combined with PD-1 antibody, Zol combined with PD-1 antibody had a synergistic antitumor effect (Figure 14A). Because Zol's bisphosphonic acid head P-C-P is highly hydrophilic, it is mainly bound to bone. Therefore, the TH-Z 93 compound was designed in this laboratory by retaining the bisphosphonic acid head P-C-P structure of the active site and adding fat-soluble groups. We combined TH-Z 93 with PD-1 antibody, and Sim with PD-1 antibody to evaluate the therapeutic effect on MC38 subcutaneously transplanted tumors in mice. Figure 14B shows that the combinations of TH-Z 93 with PD-1 antibody, and Sim with PD-1 antibody have synergistic antitumor effect.

In a mice model of in situ 4T1 breast cancer , in-vivo imaging of mice including IVIS Spectrum and Quantum FX microCT showed that the two combination have a good effect on inhibiting in situ Luci-4T1 breast cancer and its metastasis (Figures 15A, 15B). Regarding the suppression of the lung metastasis of the breast cancer, at the end point of the experiment, after the lungs of each group were isolated and immersed in picric acid, metastases could be seen with naked eyes (Figure 16A). The number of metastatic foci in lung, liver, and kidney is shown in Figure 16B. Figure 16C is the survival curve of the mice in each group after the administration, and the combination index is calculated: TH-Z 93 + PD-1, survival rate, CDI = 0.9, Sim + PD-1, survival rate, CDI = 0.78. There was no significant difference between the groups for the weight changes of mice during the administration (Figure 16D). Based on the above description, the combination of statins and bisphosphonates with immune checkpoint inhibitor PD-1 antibodies has a combined antitumor effect.

### Example 5

### Statins and bisphosphonates combined with the radiation therapy have combined anti-tumor effects

Materials and Sources: PLV-Luci-GFP, Ps PAX, PMD 2G plasmids were donated by Professor Du Yanan of Tsinghua University; luciferin was purchased from SYNCHEM; IVIS Spectrum in-vivo imaging system was purchased from PerkinElmer. X-Ray irradiator was purchased from RadSource.

Method: 1x10⁶ 4T1 cells were inoculated subcutaneously into the left hip of 6-week-old female BALB/c mice, and the tumor volume was measured using a vernier caliper, and the tumor volume was calculated using the formula of length (mm) × width² (mm)/2. Grouping and administration and the irradiation treatment were started until the tumor grew to the 7th day. Dosing or irradiation regimen: Zol administration group, 1 mg/kg, once every 6 days for a total of 4 times; Sim administration group, 2 mg/kg, once every two days for a total of 12 times; the irradiation group, at a dose of 6 Gy/mouse, and the irradiation was performed on the 7th and 19th day after tumor transplantation. The mice were exposed the tumor site for irradiation only. On the 31st day, mice were injected with potassium luciferin for in-vivo imaging, and the tumors of the mice were taken out for photograph recording.

Previous studies have found that Sim and Zol can induce immunogenic cell death of tumor cells, and can promote the body's immune response to resist tumors, and the radiation therapy is also currently believed to induce immunogenic cell death. From this point we speculate that the combination of Sim and Zol with the radiation therapy may have a synergistic antitumor effect. We established a subcutaneous model of 4T1 breast cancer in mice, and then performed Sim + the radiation therapy, and Zol + the radiation therapy treatments. The specific dosing regimen of the experiment is shown in Figure 17A. After a certain period of dosing, the mice were injected with potassium luciferin for in-vivo imaging recording to record the luminescence value, and the tumor tissue was taken out for photo recording. It can be seen from the figure that the tumor volume in the Zol, Sim and the radiation therapy alone group was significantly reduced compared with the control group (Figures 17B, 17C), while the combined group of Zol and the radiation therapy, Sim + the radiation therapy have a significantly synergistic antitumor effect compared with the control and single drug groups, (Figure 17D).

As shown in Figure 17, (Figure 17A) shows the experimental flowchart. (Figure 17B) In-vivo imaging of mice (left) and tumor size images (right) treated by Zol combined with the radiation therapy. (Figure 17C) In-vivo imaging of mice (left) and tumor size images (right) treated by Sim combined with the radiation therapy. (Figure 17D) Tumor volume at the end of the experiment after treatment by Zol and Sim combined with the radiation therapy (on day 31 after the tumor transplantation). Zol + R, CDI = 0.73, Sim + R, CDI = 0.79.

### Example 6

### Statins and bisphosphonates combined with the chemotherapeutic drug 5-FU have synergistic antitumor effects

Materials and Sources: BALB/c mice were purchased from Beijing Charles RiverCo., Ltd., 5-FU was purchased from Energy Chemical.

Method: A mouse subcutaneous tumor model of 4T1 cells was established. In the combination chemotherapy group, grouping and administarion and 5-FU treatment were started on the 9^{th} day after tumor transplantation. The experiment included 6 groups. Dosing regimen: control group; Sim group; Zol group; 5-FU group; Sim + 5-FU group; Zol + 5-FU group. Dosing regimen: Zol administration group, dissolved in sterile PBS, 1 mg/kg, once every 6 days for a total of 3 times; Sim administration group, dissolved in 75% DMSO, 2 mg/kg, once every two days for a total of 7 times; 5-FU, 50 mg/kg, administered once in total (Zol or Sim was injected 24 hours after 5-FU administration), Sim + 5-FU group, dosing of Sim, 2 mg/kg, once every two days for a total of 7 times, dosing ot 5-FU, 50 mg/kg for administering once (Sim was injected 24 h after 5-FU administration); Zol + 5-FU group, dosing of Zol, 1 mg/kg, once every 6 days for a total of 3 times, dosing of 5-FU, 50 mg/kg for administering once (Zol was injected 24 hours after 5-FU administration).

As a pyrimidine analog, 5-FU can be incorporated into the DNA and RNA synthesis, thereby inhibiting the nucleotide synthetic enzyme thymidylate synthase (TS), which ultimately leads to the cycle arrest and apoptosis of tumor cells. (5-Fluorouracil: mechanisms of action and clinical strategies, Nature Reviews Cancer 3, 330-338). From the results in Figure 18, the combination index of the two drugs is as follows: Sim + 5-FU, CDI = 0.82, Zol + 5-FU, CDI = 0.79. Therefore, Sim and Zol combined with the chemotherapeutic drug 5-FU have a synergistic antitumor effect.

### Preparation of compounds

The present disclosure relates to the preparation of compounds, which were synthesized by referring to the operations described in the international patent application WO2017041720.

In summary, the present disclosure relates to the following technical solutions:
1. Use of a mevalonate pathway inhibitor in the manufacture of a medicament for the treatment and/or prevention of tumors.
2. Use of a mevalonate pathway inhibitor in the manufacture of a medicament for inducing immunogenic cell death of tumor cells to induce immune responses.
3. Use of a mevalonate pathway inhibitor in the manufacture of a medicament for activating T lymphocytes to promote the immunity of a host body.
4. Use of a mevalonate pathway inhibitor in the manufacture of a medicament for activating DC cells to promote the immunity of a host body.
5. A method of treating and/or preventing a tumor, comprising administering a mevalonate pathway inhibitor to a subject.
6. A method of inducing immunogenic cell death of tumor cells to induce immune responses, comprising administering a mevalonate pathway inhibitor.
7. A method of activating T lymphocytes to promote the immunity in a host, comprising administering a mevalonate pathway inhibitor.
8. A method of activating DC cells to promote the immunity in a host, comprising administering a mevalonate pathway inhibitor.
9. A mevalonate pathway inhibitor, for use in the treatment and/or prevention of tumors.
10. A mevalonate pathway inhibitor, for use in causing immunogenic cell death of tumor cells to induce immune responses.
11. A mevalonate pathway inhibitor, for use in activating T lymphocytes to promote the immunity of the host body.
12. A mevalonate pathway inhibitor, for use in activating DC cells to promote the immunity of the host body.
13. The use, method or mevalonate pathway inhibitor according to any one of technical solutions 1-12, wherein the mevalonate pathway inhibitor is selected from the group consisting of acetoacetyl-CoA transferase inhibitors, HMG-CoA synthase inhibitors, HMG-CoA reductase inhibitors, mevalonate kinase inhibitors, phosphonomevalonate kinase inhibitors, mevalonate-5-pyrophosphate decarboxylase inhibitors, isopentenyl-diphosphate isomerase inhibitors, farnesyl diphosphate synthase inhibitors, geranylgeranyl diphosphate synthase inhibitors, and geranylgeranyl transferase (I, II) inhibitors.

In an alternative aspect, wherein the mevalonate pathway inhibitor is a HMG-CoA reductase inhibitor; preferably, wherein the HMG-CoA reductase inhibitor is a statin.

Preferably, the statin is selected from pravastatin, atorvastatin, rosuvastatin, fluvastatin, pitavastatin, mevastatin, lovastatin, simvastatin, cerivastatin, or pharmaceutically acceptable salts, esters, prodrugs, or solvates thereof.

Preferably, the statin is selected from simvastatin, lovastatin, and mevastatin, or pharmaceutically acceptable salts, esters, prodrugs, or solvates thereof.

Preferably, the statin is simvastatin or pharmaceutically acceptable salts, esters, prodrugs, or solvates thereof.

In another alternative aspect, wherein the mevalonate pathway inhibitor is a farnesyl diphosphate synthase inhibitor.

Preferably, the farnesyl diphosphate synthase inhibitor is a bisphosphonate compound or pharmaceutically acceptable salts, esters, prodrugs, or solvates thereof.

Preferably, wherein the bisphosphonate compound is selected from the group consisting of zoledronic acid, pamidronic acid, alendronic acid, ibandronic acid, neridronic acid, risedronic acid, olpadronic acid, minodronic acid or a compound of formula I or a pharmaceutically acceptable salt, ester, prodrug, or solvate thereof: 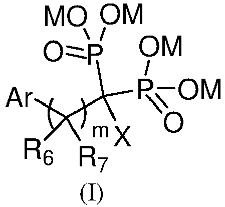 in the Formula I, the compound has a molecular weight of less than 1000, and Ar is a benzimidazolyl-type group, or an aza-benzimidazolyl group;
X is selected from the group consisting of hydrogen, hydroxy, aliphatic group, mercapto, halogen, alkoxy and alkyl; each M is independently selected from the group consisting of negative charge, hydrogen, alkyl, aliphatic group, -(CH₂)ₚ₋O-CO-R, -(CH₂)ₚ-CO-R and positive ion; wherein p is an integer of 1 to 6, R is hydrogen, alkyl or aryl; the positive ion is Li⁺, Na⁺, K⁺, Ca²⁺, Mg²⁺, NH₄⁺ or N(R')₄⁺, wherein R' is alkyl; R₆ and R₇ are each independently selected from the group consisting of hydrogen, hydroxy, mercapto, halogen, amino, aliphatic group and alkyl; m is an integer of 1 to 6.

Preferably, wherein the compound of formula I is a compound of the following formula or a pharmaceutically acceptable salt, ester, prodrug, or solvate thereof: 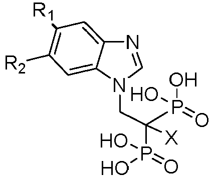 wherein R₁ and R₂ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl; X is selected from the group consisting of hydrogen, hydroxy, mercapto, and halogen.

Preferably, wherein the compound of formula I is a compound of the following formula or a pharmaceutically acceptable salt, ester, prodrug, or solvate thereof: 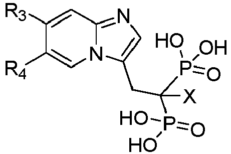 wherein R₃ and R₄ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl; X is selected from the group consisting of hydrogen, hydroxy, mercapto, and halogen.

Preferably, wherein the compound of formula I is a compound of the following formula or a pharmaceutically acceptable salt, ester, prodrug, or solvate thereof: 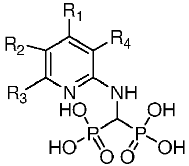 wherein:
R₁ is selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl;
R₂ is selected from the group consisting of hydrogen, alkyl, alkoxy, alkylamino, alkylthio, halogen, hydroxy, cycloalkyl, heterocyclyl, aryl and heteroaryl;
R₃ is selected from the group consisting of hydrogen, alkyl, alkoxy, alkylamino, alkylthio, halogen, hydroxy, cycloalkyl, heterocyclyl, aryl and heteroaryl;
or R₂ and R₃ together with the carbon atom to which they are attached form an aromatic or heteroaromatic ring; and
R₄ is selected from the group consisting of hydrogen, alkyl, alkoxy, alkylamino, alkylthio, halogen, hydroxy, cycloalkyl, heterocyclyl, aryl and heteroaryl.

Preferably, wherein the compound of formula I is a compound of the following formula or a pharmaceutically acceptable salt, ester, prodrug, or solvate thereof: 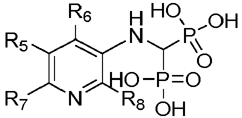 wherein
R₅ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R6 is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₇ is selected from the group consisting of hydrogen, alkyl, alkenyl, alkynyl, alkoxy, alkylamino, alkylthio, halogen, hydroxy, cycloalkyl, heterocyclyl, aryl and heteroaryl; and
R₈ is selected from the group consisting of hydrogen, alkyl, alkenyl, alkynyl, alkoxy, alkylamino, alkylthio, halogen, hydroxy, cycloalkyl, heterocyclyl, aryl and heteroaryl.

Preferably, wherein said compound of formula I is selected from:

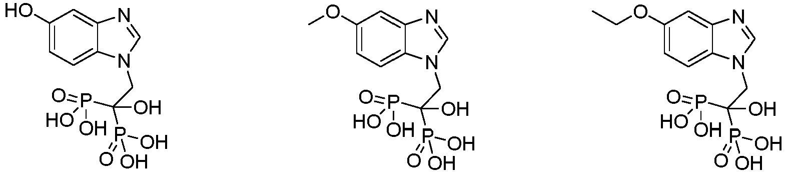

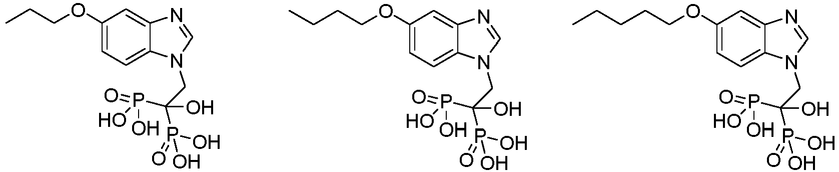

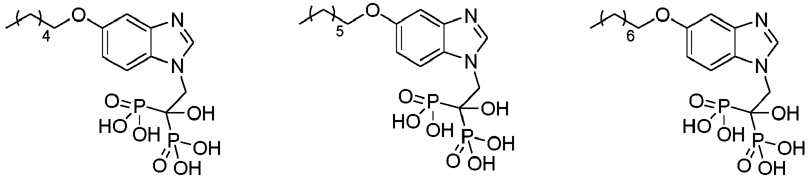

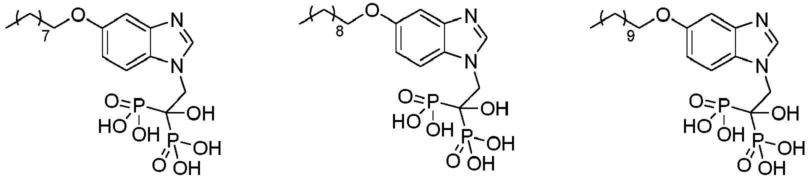

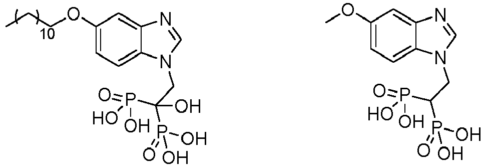

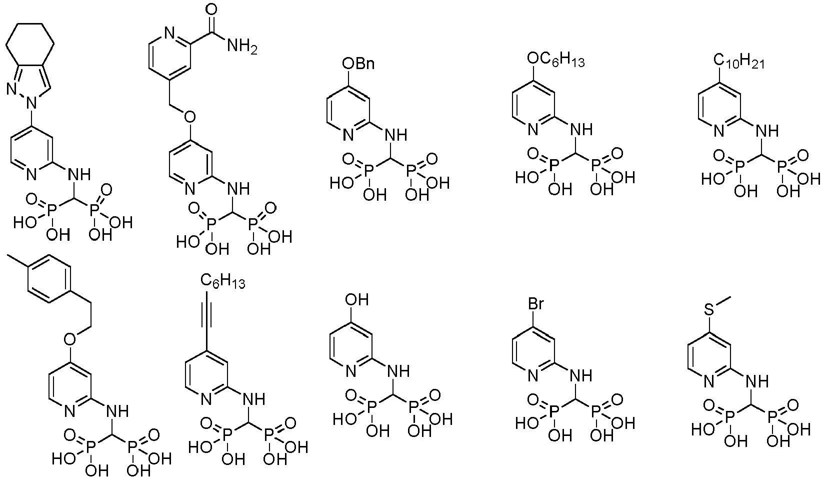

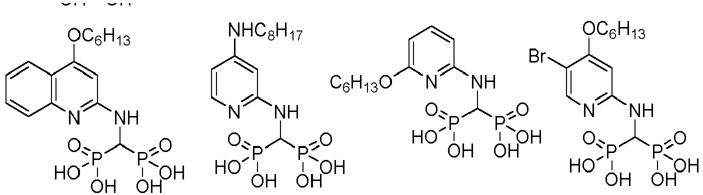

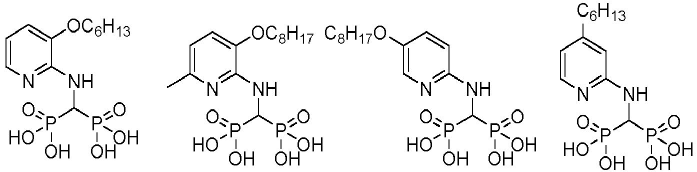

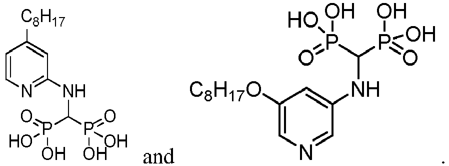

In another alternative aspect, wherein the mevalonate pathway inhibitor is a geranylgeranyl diphosphate synthase inhibitor.

Preferably, the geranylgeranyl diphosphate synthase inhibitor is a compound of the following formula or a pharmaceutically acceptable salt, ester, prodrug, or solvate thereof: 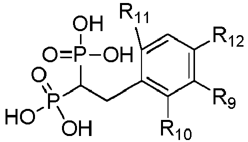 wherein
R₉ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₁₀ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₁₁ is selected from the group consisting of hydrogen, alkyl, alkenyl, alkynyl, alkoxy, alkylamino, alkylthio, halogen, hydroxy, cycloalkyl, heterocyclyl, aryl and heteroaryl; and
R₁₂ is selected from the group consisting of hydrogen, alkyl, alkenyl, alkynyl, alkoxy, alkylamino, alkylthio, halogen, hydroxy, cycloalkyl, heterocyclyl, aryl and heteroaryl.

Preferably, wherein the geranylgeranyl diphosphate synthase inhibitor is selected from: 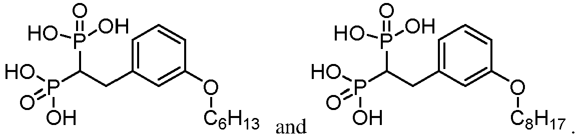
14. A pharmaceutical composition comprising a mevalonate pathway inhibitor and an antitumor drug (such as an immune checkpoint inhibitor and any drug capable of causing neo-antigen production).
15. The pharmaceutical composition according to technical solution 14, wherein the mevalonate pathway inhibitor is selected from the group consisting of acetoacetyl-CoA transferase inhibitors, HMG-CoA synthase inhibitors, HMG-CoA reductase inhibitors, mevalonate kinase inhibitors, phosphonomevalonate kinase inhibitors, mevalonate-5-pyrophosphate decarboxylase inhibitors, isopentenyl-diphosphate isomerase inhibitors, farnesyl diphosphate synthase inhibitors, geranylgeranyl diphosphate synthase inhibitors and geranylgeranyl transferase (I, II) inhibitors.

In another alternative aspect, wherein the mevalonate pathway inhibitor is a HMG-CoA reductase inhibitor; preferably, wherein the HMG-CoA reductase inhibitor is a statin.

Preferably, the statin is selected from pravastatin, atorvastatin, rosuvastatin, fluvastatin, pitavastatin, mevastatin, lovastatin, simvastatin, cerivastatin, or their pharmaceutically acceptable salts, esters, prodrugs, or solvates.

Preferably, the statin is selected from simvastatin, lovastatin, and mevastatin, or pharmaceutically acceptable salts, esters, prodrugs, or solvates thereof.

Preferably, the statin is simvastatin or a pharmaceutically acceptable salt, ester, prodrug, or solvate thereof.

In another alternative aspect, wherein the mevalonate pathway inhibitor is a farnesyl diphosphate synthase inhibitor.

Preferably, the farnesyl diphosphate synthase inhibitor is a bisphosphonate compound or a pharmaceutically acceptable salt, ester, prodrug, or solvate thereof.

Preferably, wherein the bisphosphonate compound is selected from the group consisting of zoledronic acid, pamidronic acid, alendronic acid, ibandronic acid, neridronic acid, risedronic acid, olpadronic acid, minodronic acid or a compound of formula I or a pharmaceutically acceptable salt, ester, prodrug, or solvate thereof: 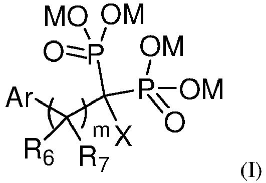 in the Formula I, the compound has a molecular weight of less than 1000, and Ar is a benzimidazolyl-type group, or an aza-benzimidazolyl group;
X is selected from the group consisting of hydrogen, hydroxy, aliphatic group, mercapto, halogen, alkoxy and alkyl; each M is independently selected from the group consisting of negative charge, hydrogen, alkyl, aliphatic group, -(CH₂)ₚ₋O-CO-R, -(CH₂)ₚ-CO-R and positive ion; wherein p is an integer of 1 to 6, R is hydrogen, alkyl or aryl; the positive ion is Li⁺, Na⁺, K⁺, Ca²⁺, Mg²⁺, NH₄⁺ or N(R')₄⁺, wherein R' is alkyl; R₆ and R₇ are each independently selected from the group consisting of hydrogen, hydroxy, mercapto, halogen, amino, aliphatic group and alkyl; m is an integer of 1 to 6.

Preferably, wherein the compound of formula I is a compound of the following formula or a pharmaceutically acceptable salt, ester, prodrug, or solvate thereof: 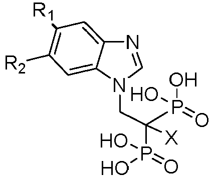 wherein R₁ and R₂ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl; X is selected from the group consisting of hydrogen, hydroxy, mercapto, and halogen.

Preferably, wherein the compound of formula I is a compound of the following formula or a pharmaceutically acceptable salt, ester, prodrug, or solvate thereof: 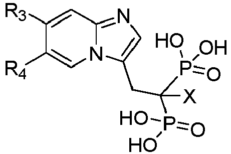 wherein R₃ and R₄ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl; X is selected from the group consisting of hydrogen, hydroxy, mercapto, and halogen.

Preferably, wherein the compound of formula I is a compound of the following formula or a pharmaceutically acceptable salt, ester, prodrug, or solvate thereof: 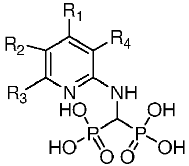 wherein
R₁ is selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl;
R₂ is selected from the group consisting of hydrogen, alkyl, alkoxy, alkylamino, alkylthio, halogen, hydroxy, cycloalkyl, heterocyclyl, aryl and heteroaryl;
R₃ is selected from the group consisting of hydrogen, alkyl, alkoxy, alkylamino, alkylthio, halogen, hydroxy, cycloalkyl, heterocyclyl, aryl and heteroaryl;
or R₂ and R₃ together with the carbon atom to which they are attached form an aromatic or heteroaromatic ring; and
R₄ is selected from the group consisting of hydrogen, alkyl, alkoxy, alkylamino, alkylthio, halogen, hydroxy, cycloalkyl, heterocyclyl, aryl and heteroaryl.

Preferably, wherein the compound of formula I is a compound of the following formula or a pharmaceutically acceptable salt, ester, prodrug, or solvate thereof: 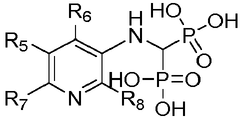 wherein:
R₅ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R6 is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₇ is selected from the group consisting of hydrogen, alkyl, alkenyl, alkynyl, alkoxy, alkylamino, alkylthio, halogen, hydroxy, cycloalkyl, heterocyclyl, aryl and heteroaryl; and
R₈ is selected from the group consisting of hydrogen, alkyl, alkenyl, alkynyl, alkoxy, alkylamino, alkylthio, halogen, hydroxy, cycloalkyl, heterocyclyl, aryl and heteroaryl.

Preferably, wherein said compound of formula I is selected from:

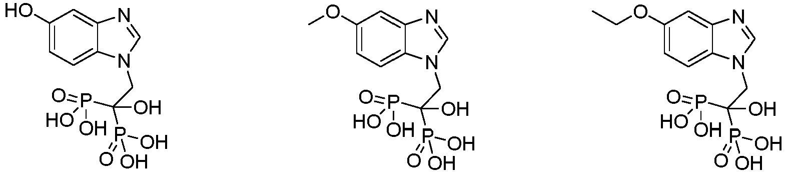

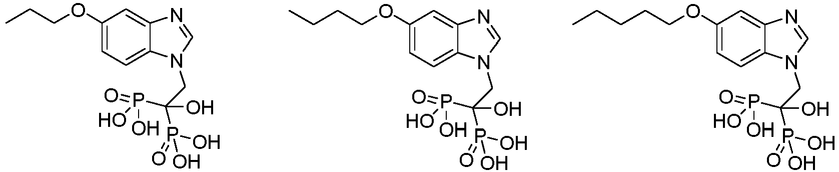

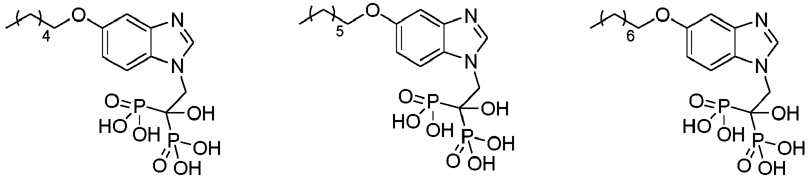

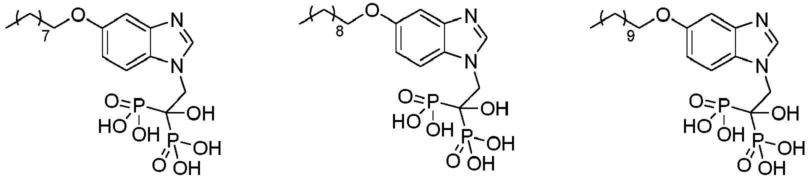

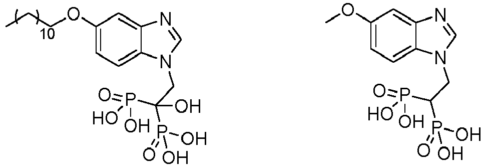

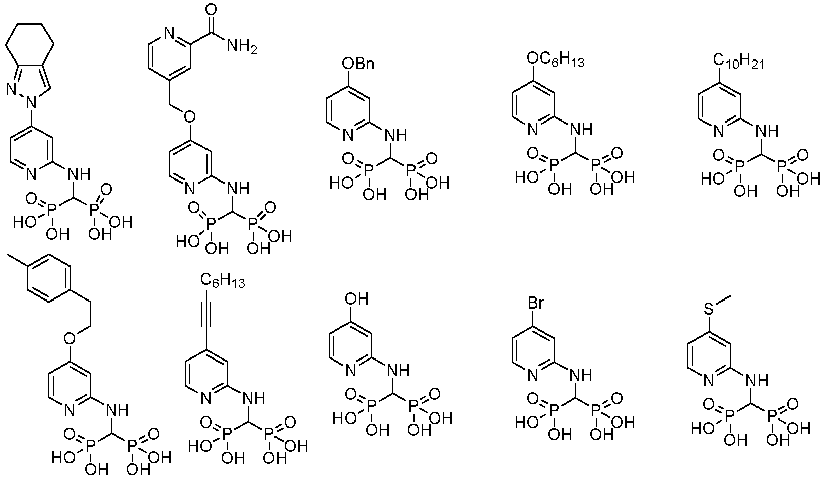

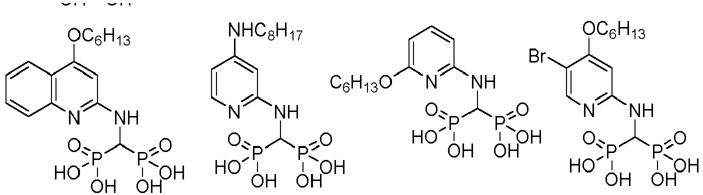

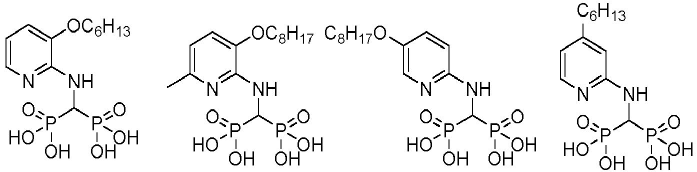

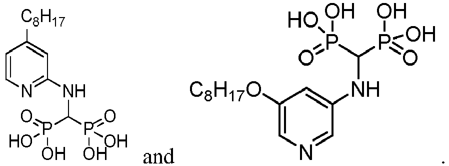

In another alternative aspect, wherein the mevalonate pathway inhibitor is a geranylgeranyl diphosphate synthase inhibitor.

Preferably, the geranylgeranyl diphosphate synthase inhibitor is a compound of the following formula or a pharmaceutically acceptable salt, ester, prodrug, or solvate thereof: 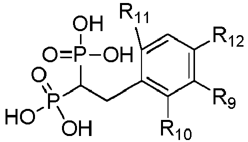 wherein
R₉ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₁₀ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₁₁ is selected from the group consisting of hydrogen, alkyl, alkenyl, alkynyl, alkoxy, alkylamino, alkylthio, halogen, hydroxy, cycloalkyl, heterocyclyl, aryl and heteroaryl; and
R₁₂ is selected from the group consisting of hydrogen, alkyl, alkenyl, alkynyl, alkoxy, alkylamino, alkylthio, halogen, hydroxy, cycloalkyl, heterocyclyl, aryl and heteroaryl.

Preferably, wherein the geranylgeranyl diphosphate synthase inhibitor is selected from: 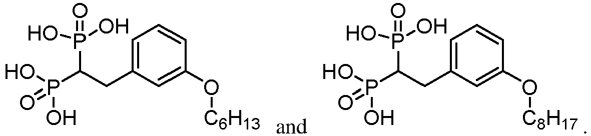
16. The pharmaceutical composition according to any one of technical solutions 14-15, wherein the immune checkpoint inhibitor is selected from the group consisting of CTLA-4 antibody, PD-1 antibody, PD-L1 antibody or PD-L2 fusion protein, IDO inhibitor, OX-40 agonist, CD137 antibody, IL-2, a-CD27/CD70 antibody, TGF-β antibody, TIM-3 antibody, LAG-3 antibody, and BTLA antibody; preferably, the immune checkpoint inhibitor is selected From Ipilimumab, Pembrolizumab, Nivolumab, Pidilizumab, BMS-956559, MPDL3280A, MEDI4736, MSB0010718C, AMP-224, CTLA-4 antibody, PD-1 antibody, and PDL-1; preferably, the immune checkpoint inhibitor is a PD-1 antibody.
17. The pharmaceutical composition according to any one of technical solutions 14-15, wherein the drug capable of causing neo-antigen production is selected from 5-FU, 5-azacytidine, 5-aza-2'-deoxycytidine, thiopurine, thioguanine, nelarabine, cladribine, clofarabine, capecitabine, cytarabine, ancitabine, gemcitabine, troxacitabine, decitabine; doxorubicin, mitoxantrone, daunorubicin, epirubicin and idarubicin; oxaliplatin, enenloplatin, carboplatin, nedaplatin, dicycenloplatin, cisplatin; bortezomib, and cetuximab, preferably, any drug capable of causing neo-antigen production is 5-FU, azacytidine, deoxycytidine, doxorubicin, mitoxantrone, oxaliplatin, bortezomib, or cetuximab.
18. Use of a mevalonate pathway inhibitor and an antitumor drug (such as immune checkpoint inhibitors and any drug capable of causing neo-antigen production) in the manufacture of a medicament for the treatment of tumors.
19. A combination comprising (1) a mevalonate pathway inhibitor, and (2) an antitumor drug (such as an immune checkpoint inhibitor and any drug capable of causing neo-antigen production) or the radiation therapy.
20. A method of treating a tumor, said method comprising administering to a subject in need thereof: (1) a mevalonate pathway inhibitor, and (2) an antitumor drug (such as an immune checkpoint inhibitor and any drug capable of causing neo-antigen production) or the radiation therapy.
21. A mevalonate pathway inhibitor for use in combination with an antitumor drug (such as immune checkpoint inhibitors and any drug capable of causing neo-antigen production) or the radiation therapy, for use in the treatment of tumors.
22. A method of treating a tumor, said method comprising infusing DC cells into a subject in need thereof, wherein said DC cells have been treated with a mevalonate pathway inhibitor; preferably, said DC cells are donor DC cells; preferably, the DC cells are autologous DC cells.
23. The use of technical solution 18 or the combination of technical solution 19 or the method of technical solution 20, 22 or 23 or the mevalonate pathway inhibitor of technical solution 21, wherein said mevalonate pathway inhibitor is selected from acetoacetyl-CoA transferase inhibitors, HMG-CoA synthase inhibitors, HMG-CoA reductase inhibitors, mevalonate kinase inhibitors, phosphonomevalonate kinase inhibitors, mevalonate-5-pyrophosphate decarboxylase inhibitors, isopentenyl-diphosphate isomerase inhibitors, farnesyl diphosphate synthase inhibitors, geranylgeranyl diphosphate synthase inhibitors and geranylgeranyl transferase (I, II) Inhibitors.

In another alternative aspect, wherein the mevalonate pathway inhibitor is a HMG-CoA reductase inhibitor; preferably, wherein the HMG-CoA reductase inhibitor is a statin.

Preferably, the statin is selected from pravastatin, atorvastatin, rosuvastatin, fluvastatin, pitavastatin, mevastatin, lovastatin, simvastatin, or cerivastatin, or their pharmaceutically acceptable salts, esters, prodrugs, or solvates.

Preferably, the statin is selected from simvastatin, lovastatin, and mevastatin, or pharmaceutically acceptable salts, esters, prodrugs, or solvates thereof.

Preferably, the statin is simvastatin or a pharmaceutically acceptable salt, ester, prodrug, or solvate thereof.

In another alternative aspect, wherein the mevalonate pathway inhibitor is a farnesyl diphosphate synthase inhibitor.

Preferably, the farnesyl diphosphate synthase inhibitor is a bisphosphonate compound or a pharmaceutically acceptable salt, ester, prodrug, or solvate thereof.

Preferably, wherein the bisphosphonate compound is selected from the group consisting of zoledronic acid, pamidronic acid, alendronic acid, ibandronic acid, neridronic acid, risedronic acid, olpadronic acid, minodronic acid or a compound of formula I or a pharmaceutically acceptable salt, ester, prodrug, or solvate thereof: 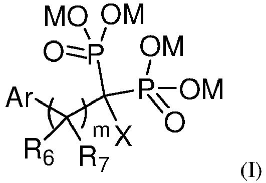 in the Formula I, the compound has a molecular weight of less than 1000, and Ar is a benzimidazolyl-type group, or an aza-benzimidazolyl group;
X is selected from the group consisting of hydrogen, hydroxy, aliphatic group, mercapto, halogen, alkoxy and alkyl; each M is independently selected from the group consisting of negative charge, hydrogen, alkyl, aliphatic group, -(CH₂)ₚ-O-CO-R, -(CH₂)ₚ-CO-R and positive ion; wherein p is an integer of 1 to 6, R is hydrogen, alkyl or aryl; the positive ion is Li⁺, Na⁺, K⁺, Ca²⁺, Mg²⁺, NH₄⁺ or N(R')₄⁺, wherein R' is alkyl; R₆ and R₇ are each independently selected from the group consisting of hydrogen, hydroxy, mercapto, halogen, amino, aliphatic group and alkyl; m is an integer of 1 to 6.

Preferably, wherein the compound of formula I is a compound of the following formula or a pharmaceutically acceptable salt, ester, prodrug, or solvate thereof: 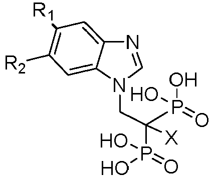 wherein R₁ and R₂ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl; X is selected from the group consisting of hydrogen, hydroxy, mercapto, and halogen.

Preferably, wherein the compound of formula I is a compound of the following formula or a pharmaceutically acceptable salt, ester, prodrug, or solvate thereof: 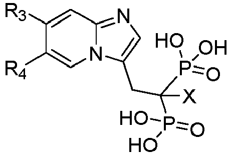 wherein R₃ and R₄ are independently selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl; X is selected from the group consisting of hydrogen, hydroxy, mercapto, and halogen.

Preferably, wherein the compound of formula I is a compound of the following formula or a pharmaceutically acceptable salt, ester, prodrug, or solvate thereof: 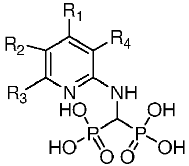 wherein
R₁ is selected from the group consisting of hydrogen, alkyl, alkenyl, alkynyl, alkoxy, alkylamino, alkylthio, halogen, hydroxy, cycloalkyl, heterocyclyl, aryl and heteroaryl, wherein the alkyl group in said alkoxy group is optionally substituted with aryl, heteroaryl or heterocyclyl, wherein said aryl, heteroaryl or heterocyclyl is optionally substituted with alkyl or carbamoyl;
R₂ is selected from the group consisting of hydrogen, alkyl, alkoxy, alkylamino, alkylthio, halogen, hydroxy, cycloalkyl, heterocyclyl, aryl and heteroaryl;
R₃ is selected from the group consisting of hydrogen, alkyl, alkoxy, alkylamino, alkylthio, halogen, hydroxy, cycloalkyl, heterocyclyl, aryl and heteroaryl;
or R₂ and R₃ together with the carbon atom to which they are attached form an aromatic or heteroaromatic ring; and
R₄ is selected from the group consisting of hydrogen, alkyl, alkoxy, alkylamino, alkylthio, halogen, hydroxy, cycloalkyl, heterocyclyl, aryl and heteroaryl.

Preferably, wherein the compound of formula I is a compound of the following formula or a pharmaceutically acceptable salt, ester, prodrug, or solvate thereof: 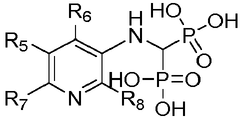 wherein:
R₅ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R6 is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₇ is selected from the group consisting of hydrogen, alkyl, alkenyl, alkynyl, alkoxy, alkylamino, alkylthio, halogen, hydroxy, cycloalkyl, heterocyclyl, aryl and heteroaryl; and
R₈ is selected from the group consisting of hydrogen, alkyl, alkenyl, alkynyl, alkoxy, alkylamino, alkylthio, halogen, hydroxy, cycloalkyl, heterocyclyl, aryl and heteroaryl.

Preferably, wherein said compound of formula I is selected from:

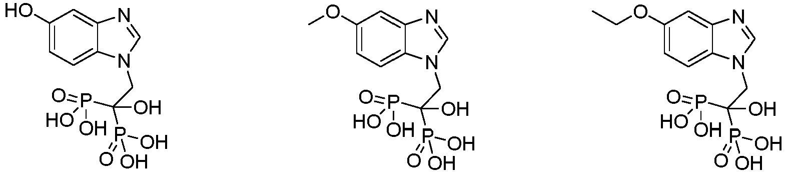

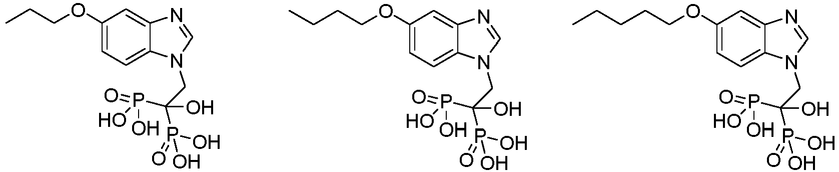

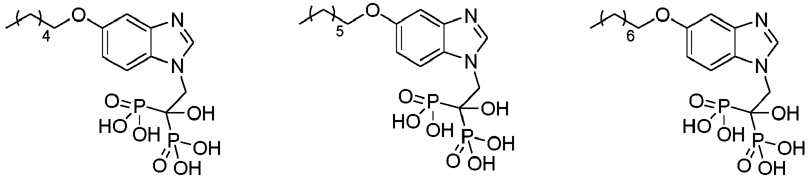

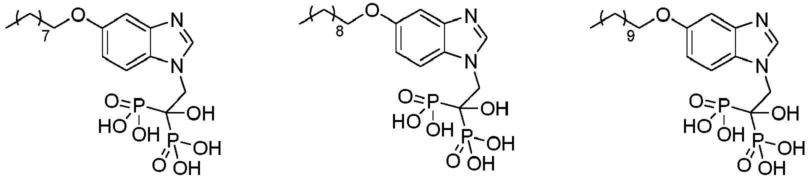

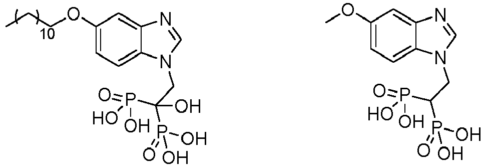

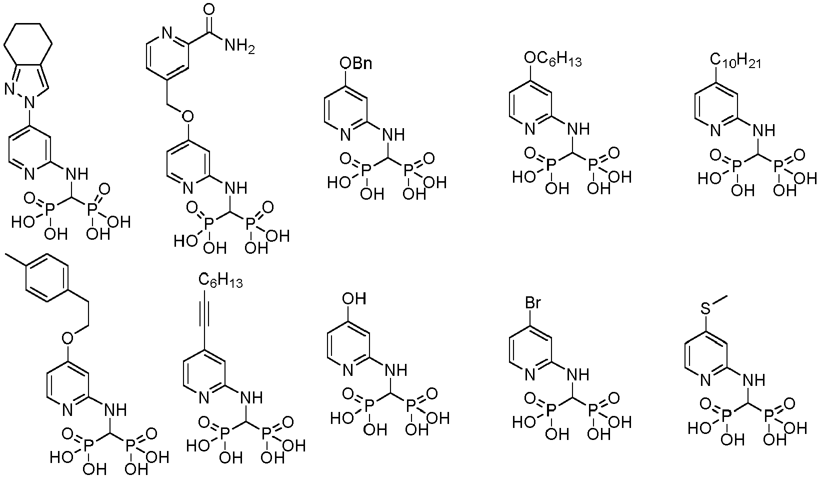

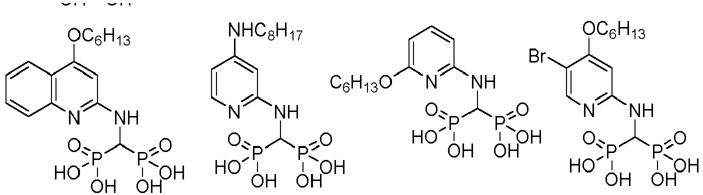

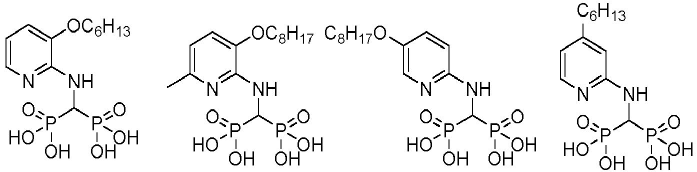

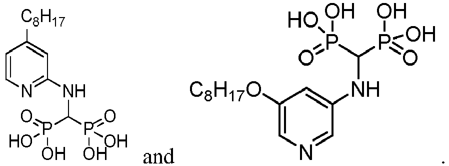

In another alternative aspect, wherein the mevalonate pathway inhibitor is a geranylgeranyl diphosphate synthase inhibitor.

Preferably, the geranylgeranyl diphosphate synthase inhibitor is a compound of the following formula or a pharmaceutically acceptable salt, ester, prodrug, or solvate thereof: 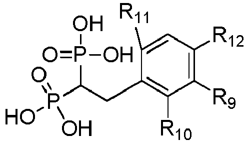 wherein
R₉ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₁₀ is selected from the group consisting of hydrogen, alkyl, alkenyl, alkynyl, alkoxy, alkylamino, alkylthio, halogen, hydroxy, cycloalkyl, heterocyclyl, aryl and heteroaryl;
R₁₁ is selected from the group consisting of hydrogen, alkyl, alkenyl, alkynyl, alkoxy, alkylamino, alkylthio, halogen, hydroxy, cycloalkyl, heterocyclyl, aryl and heteroaryl; and
R₁₂ is selected from the group consisting of hydrogen, alkyl, alkenyl, alkynyl, alkoxy, alkylamino, alkylthio, halogen, hydroxy, cycloalkyl, heterocyclyl, aryl and heteroaryl.

Preferably, wherein the geranylgeranyl diphosphate synthase inhibitor is selected from: 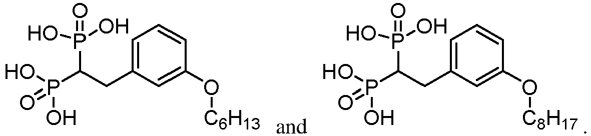
24. The use or the combination or the method or the mevalonate pathway inhibitor according to any one of the above technical solutions 18-23, wherein the immune checkpoint inhibitor is selected from the group consisting of CTLA-4 antibody, PD-1 antibody, PD-L1 antibody or PD-L2 fusion protein, IDO inhibitor, OX-40 agonist, CD137 antibody, IL-2, a-CD27/CD70 antibody, TGF-β antibody, TIM-3 antibody, LAG-3 antibody, BTLA antibody; preferably, the immune checkpoint inhibitor is selected from the group consisting of Ipilimumab, Pembrolizumab, Nivolumab, Pidilizumab, BMS-956559, MPDL3280A, MEDI4736, MSB0010718C, AMP-224, CTLA-4 antibody, PD-1 antibody, PDL-1; Preferably, the immune checkpoint inhibitor is a PD-1 antibody.
25. The use or the combination or the method or mevalonate pathway inhibitors according to any one of technical solutions 18-23, wherein said any drug capable of causing neo-antigen production is selected from 5-FU, 5-azacytidine, 5-aza-2'-deoxycytidine, thiopurine, thioguanine, nelarabine, cladribine, clofarabine, capecitabine, cytarabine, ancitabine, gemcitabine, troxacitabine, decitabine; doxorubicin, mitoxantrone, daunorubicin, epirubicin, and idarubicin; oxaliplatin, enloplatin, carboplatin, nedaplatin, dicycenloplatin, cisplatin; bortezomib, cetuximab, preferably, any drug capable of causing neo-antigen production is 5-FU, 5-azacytidine, deoxycytidine, doxorubicin, mitoxantrone, oxaliplatin, bortezomib, cetuximab.
26. The use or the combination or the method or mevalonate pathway inhibitors according to any of technical solutions 18-25, wherein the tumor is selected from the group consisting of breast cancer, prostate cancer, interstitial cell-like cancer, hepatocellular carcinoma, colorectal cancer, multiple myeloma, lung cancer, non-small cell lung cancer, lymphoma, melanoma, neuroblastoma, malignant epithelioma, renal cell carcinoma, blood tumor, bladder cancer, head and neck cancer, gastrointestinal cancer, chronic lymphocytic leukemia (CLL), acute lymphocytic leukemia (ALL), diffuse large B-cell lymphoma, cervical cancer, synovial sarcoma, neuroblastoma.
27. Activated DC cells, which have been treated with a mevalonate pathway inhibitor, wherein the mevalonate pathway inhibitor is as described in technical solution 13.
28. A tumor vaccine comprising activated DC cells, which have been treated with a mevalonate pathway inhibitor, wherein the mevalonate pathway inhibitor is as described in technical solution 13.

This application refers to various issued patents, published patent applications, journal articles, and other publications, all of which are incorporated herein by reference. If there is a conflict between the reference cited and this specification, this specification shall prevail. Furthermore, any particular embodiment of the disclosure falling within the scope of the prior art may be explicitly excluded from any one or more claims. Because the embodiments are considered to be known to a person skilled in the art, they can be excluded even if the exclusion is not explicitly listed in this application. Any particular embodiment of the disclosure may be excluded from any claim for any reason, whether or not it is related to the existence of prior art.

Using only routine experimentation, one of ordinary skill in the art will recognize, or be able to determine many equivalents to the specific embodiments described herein. The scope of the embodiments of the present disclosure described herein is not intended to be limited to the above description, but rather as set forth in the appended claims. It will be understood by those skilled in the art that various changes and modifications to the description can be made without departing from the spirit or scope of the invention as defined by the claims.

## Claims

1. Use of a mevalonate pathway inhibitor in the manufacture of a medicament for the treatment and/or prevention of tumors.

2. Use of a mevalonate pathway inhibitor in the manufacture of a medicament for inducing immunogenic cell death of tumor cells to induce an immune response.

3. Use of a mevalonate pathway inhibitor in the manufacture of a medicament for activating T lymphocytes to promote the immunity of a host body.

4. Use of a mevalonate pathway inhibitor in the manufacture of a medicament for activating DC cells to promote the immunity of a host body.

5. A method of treating and/or preventing a tumor, comprising administering a mevalonate pathway inhibitor to a subject.

6. A method of inducing an immune response to induce immunogenic cell death of tumor cells, comprising administering a mevalonate pathway inhibitor.

7. A method for activating T lymphocytes to promote the immunity in a host, comprising administering a mevalonate pathway inhibitor.

8. A method for activating DC cells to promote the host immunity, comprising administering a mevalonate pathway inhibitor.

9. A mevalonate pathway inhibitor, for use in the treatment and/or prevention of tumors.

10. A mevalonate pathway inhibitor, for use in causing immunogenic cell death of tumor cells to induce an immune response.

11. A mevalonate pathway inhibitor, for use in activating T lymphocytes to promote the immunity of a host body.

12. A mevalonate pathway inhibitor, for use in activating DC cells to promote the immunity of a host body.
